# EUROPEAN PATENT SPECIFICATION

(11) **EP 3 030 902 B1**
(45) Date of publication and mention of the grant of the patent: **25.09.2019**
(21) Application number: 14780581.6
(22) Date of filing: 05.08.2014
(51) Int. Cl.: A61K 31/4375, A61K 31/444, A61K 31/496, A61K 31/713, C12N 5/0793, G01N 33/50

(54) **NEW SCREENING METHOD FOR THE TREATMENT FRIEDREICH'S ATAXIA**
NEUES SCREENING-VERFAHREN ZUR BEHANDLUNG VON FRIEDREICH-ATAXIE
NOUVEAU PROCÉDÉ DE CRIBLAGE POUR TRAITER L'ATAXIE DE FRIEDREICH

(30) Priority: 07.08.2013 EP 13179587
(43) Date of publication of application: 15.06.2016
(73) Proprietor: Friedrich Miescher Institute for Biomedical Research, 4058 Basel (CH); Novartis AG, 4056 Basel (CH); IRM LLC, Hamilton HM 12 (BM)
(72) Inventor: BUEHLER, Marc, CH-4125 Riehen (CH); MIRAGLIA, Loren, San Diego, California 92121 (US); MONOVICH, Lauren, Gilchrist, Cambridge, Massachusetts 02139 (US); ORTH, Anthony, San Diego, California 92121 (US); TU, Buu, San Diego, California 92121 (US)
(74) Representative: Favre, Nicolas
(86) International application number: PCT/IB2014/063708
(87) International publication number: WO 2015/019286

(56) References cited:
- WO-A1-2008/122615
- WO-A1-2011/119842
- M. Lufino: "Drug screening based on FRDA genomic-reporter fusion vectors identifies two candidate molecules able to up-regulate FXN expression", 4th International Friedreich's Ataxia Scientific Conference, May 5th-7th, 2011, 5 May 2011 (2011-05-05), XP055086966, Retrieved from the Internet: URL:http://www.curefa.org/_pdf/4thInternat ionalFAConferenceAbstracts.pdf [retrieved on 2013-11-06]
- SARSERO JOSEPH P ET AL: "UPREGULATION OF EXPRESSION FROM THE FRDA GENOMIC LOCUS FOR THE THERAPY OF FRIEDREICH ATAXIA", JOURNAL OF GENE MEDICINE, JOHN WILEY & SONS, INC, US, vol. 5, no. 1, 1 January 2003 (2003-01-01) , pages 72-81, XP009082996, ISSN: 1099-498X, DOI: 10.1002/JGM.320
- HEBERT ET AL: "Targeting the gene in Friedreich ataxia", BIOCHIMIE, MASSON, PARIS, FR, vol. 90, no. 8, 1 August 2008 (2008-08-01) , pages 1131-1139, XP027017452, ISSN: 0300-9084 [retrieved on 2007-12-28]

## Description

### Field of the invention

The present invention provides a screening method and a method for the treatment of Friedreich's ataxia.

### Background of the invention

Friedreich's Ataxia (FRDA) is autosomal recessive neurodegenerative disease caused by deficiency of the mitochondrial protein frataxin (FXN) resulting in the repression of the iron-binding protein frataxin (Pandolfo. J Neurol 256 Suppl 1, 3-8 (2009)). This deficiency results from a hyperexpansion of a trinucleotide GAA repeat in the first intron of the FXN gene Gottesfeld, J.M. Pharmacol.Ther. 116, 236-248 (2007). Reduced FXN expression provokes a spectrum of pathophysiological defects, including demyelination and loss of motor coordination. FRDA is the most common cause of inherited recessive ataxias across Europe and several epidemiological studies have estimated the prevalence of FRDA as 2-3 cases per 100,000 people in Caucasian populations (/Campuzano, V. et al. Science 271, 1423-1427 (1996); Schulz, J.B. et al. Nat Rev Neurol 5, 222-234 (2009); Durr, A. et al. N Engl J Med 335, 1169-1175 (1996)). Despite years of research, the precise mechanisms of FXN gene regulation remain poorly understood and treatment options for FRDA are limited. The long non-coding GAA triplet repeats found in intron 1 of the FXN gene in FRDA patients impedes transcription elongation. Therefore, overcoming this blockade and reactivating FXN expression is a particularly attractive therapeutic strategy. In addition, approaches that enhance transcription rates or increase FXN protein stability are also thought to be able to correct the FXN protein deficiency in FRDA patients (Kumari, D., Biacsi, R.E. & Usdin, K. R The Journal of biological chemistry 286, 4209-4215 (2011); Tsou, A.Y., Friedman, L.S., Wilson, R.B. & Lynch, D.R. CNS Drugs 23, 213-223 (2009); Sturm, B. et al. Eur J Clin Invest 35, 711-717 (2005)).

Cell-based assays are essential for the identification of druggable regulators of FXN gene expression and low molecular weight compounds that alleviate FXN deficiency. Previous attempts at developing HTS-friendly assays have used artificial intronic sequences with extended GAA tracts or FXN reporter fusions (Sarsero, J.P. et al. J Gene Med 5, 72-81 (2003); Sarsero, J.P. et al. Mamm Genome 16, 228-241 (2005); Grant, L. et al. FEBS letters 580, 5399-5405 (2006); Soragni, E. et al. Nucleic Acids Res (2008)). A major drawback of these systems is that they do not assess the activity of the endogenous FXN gene in native chromosomal and disease-relevant contexts. This limits the identification of modulators of FXN expression, because the endogenous chromatin structure and/or nuclear organization of the human genome are major determinants of FXN gene activity (Herman, D. et al. Nat Chem Biol 2, 551-558 (2006); Saveliev, A., Everett, C., Sharpe, T., Webster, Z. & Festenstein, R. Nature 422, 909-913 (2003); Castaldo, I. et al. Journal of medical genetics 45, 808-812 (2008)). There was thus a need in the art for a drug discovery platform that combines genetically accurate reporter systems, disease-relevant models, and high-throughput biology.

M. Lufino ("Drug screening based on FRDA genomic-reporter fusion vectors identifies two candidate molecules able to up-regulate FXN expression", 4th International Friedreich's Ataxia Scientific Conference, May 5th-7th, 2011, 5 May 2011 (2011-05-05), URL:http://www.curefa.org/ pdf/ 4th International FAConferenceAbstracts.pdf) provides a cell based high-throughput screening assay based on a cell model that recapitulates the GAA-mediated repression of frataxin expression linked to Friedreich's ataxia. The human clonal cell lines comprise a FRDA genomic-reporter fusion vector wherein a by Luciferase gene was inserted in-frame at the 3'-end of the FRDA gene (FRDA-Luc). The cell lines allow rapid quantification of frataxin expression by a Luciferase assay. The screening identified seven compounds which increased FRDA-Luciferase expression.

SARSERO JOSEPH P ET AL ("UPREGULATION OF EXPRESSION FROM THE FRDA GENOMIC LOCUS FOR THE THERAPY OF FRIEDREICH ATAXIA", JOURNAL OF GENE MEDICINE, JOHN WILEY & SONS, INC, US, vol. 5, no. 1, 1 January 2003 (2003-01-01), pages 72-81) discloses a sensitive cellular assay for frataxin expression from the entire human FRDA functional locus that for the identification of potentially therapeutic pharmacological agents to treat Friedreich's ataxia. Cassettes consisting of the gene encoding EGFP linked to a kanamycin/neomycin resistance were used as reporter genes. Two in-frame fusions between the FRDA gene and a gene coding for enhanced green fluorescent protein (EGFP) were constructed. One fusion is within exon 2 of the FRDA gene. The other is at the end of exon 5a, containing the entire frataxin protein fused to EGFP. Both constructs were shown to drive the expression of EGFP from the regulatory elements of the FRDA locus, with the frataxin-EGFP fusion proteins targeted to the mitochondria. Stable cell lines containing the EGFP fusion in exon 5a were produced. Enhancement of FRDA gene expression by hemin and butyric acid was demonstrated.

HEBERT ET AL ("Targeting the gene in Friedreich ataxia", BIOCHIMIE, MASSON, PARIS, FR, vol. 90, no. 8, 1 August 2008 (2008-08-01), pages 1131-1139) reviews therapeutic option to target the gene FTX (its expression) in Friedreich's ataxia. Potential treatment options involving small molecule compounds that target the GAA expanded gene by increasing the level of frataxin message and protein are discussed.

WO 2011/119842 A1 (DAVID GLADSTONE INST [US]; FINKBEINER STEVEN M [US]; RAO VIKRAM RAMNAT) 29 September 2011 (2011-09-29)) discloses agents for use in the treatment of a neurological disorder or a neurodegenerative disease such as epilepsy, ischemia, cerebellar ataxia in an individual, wherein the agent modulates activity of protein kinase D1 (P[R]KD1). The agent is selected from the group of a small molecule, an inhibitory nucleic acid (such as siRNA) or an antibody binding to protein kinase D1.

WO 2008/122615 A1 (NOVARTIS AG [CH]; DOBLER MARKUS ROLF [US]; JEWELL CHARLES FRANCIS JR[) 16 October 2008 (2008-10-16)) describes [2,6] Naphthyridine of formula (I) as inhibitors of PRKD1.

### Summary of the invention

In order to address the needs in the art, the present inventors generated a reporter cell line of human origin, characterized in that a reporter gene has been fused to the 3'-end of the endogenous frataxin (FTX) gene, wherein said reporter cell line is compatible with high-throughput biology and enables accurate monitoring of endogenous FXN gene expression. Using this cell line, the present inventors have been able to identify a new physiologically-relevant link between PKD and FRDA and showed for the first time that PKD inhibitors are agents which can be used to treat capable FRDA.

The present invention hence encompasses an agent which modulate the activity of the *PRKD1* gene product, protein kinase D1, for use in the treatment of Friedreich's ataxia as defined in the claims. In some embodiments, the protein kinase D1 inhibitor is a [2,6] Naphtyhyridine of formula (I): 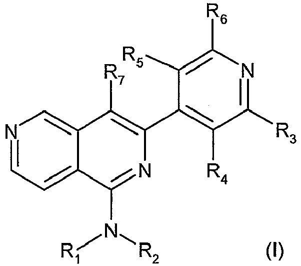 wherein
R₁ and R₂ are independently hydrogen, alkyl, cycloalkyl, heterocyclyl, each of which is optionally substituted by one to two R₈, wherein R₈ is hydrogen, halogen, alkyl, R₉-O--, (R₁₀)(R₁₁)N--, (R₁₂)(R₁₃)N-C(O)--, aryl, or heterocyclyl or heteroaryl, said heterocyclyl and heteroaryl are optionally substituted by one or two alkyl groups;
R₁ and R₂ taken together with the nitrogen atom to which they are attached to optionally form a 4-7 membered ring;
R₃ is (R₁₄)(R₁₅)N--, or halogen;
R₄, R₅, R₆ and R₇ are independently hydrogen, halogen, alkyl, (C₃-C₇) cycloalkyl, aryl-alkyl, aryl, or alkoxy;
R₉, R₁₀, R₁₁, R₁₂ and R₁₃ are independently hydrogen, alkyl-O-C(O)--, alkyl-NH-C(O)--, alkyl-C(O)-NH-C(O)--, cycloalkyl, cycloalkyl-alkyl--, R₁₆-SO₂--, R₁₇-C(O)--, heterocyclyl or alkyl, said heterocyclyl is further optionally substituted by one or two cycloalkyl-alkyl-- groups, and said alkyl is further optionally substituted by one or two groups selected from hydroxy, alkoxy, alkylamine, dialkylamine, or heteroaryl;
R₁₀ and R₁₁ taken together with the nitrogen atom to which they are attached to optionally form a 5-7 membered ring;
R₁₂ and R₁₃ taken together with the nitrogen atom to which they are attached to optionally form a 5-7 membered ring;
R₁₄ and R₁₅ are independently hydrogen, alkyl, aryl, cycloalkyl, aryl-alkyl--, heterocyclyl or heteroaryl, said alkyl, cycloalkyl, aryl and heteroaryl are further optionally substituted by one or two groups selected from alkyl, alkoxy, hydroxy, halogen, haloalkyl, cyano, or R₁₈-NH-C(O)--;
R₁₆ is aryl or heteroaryl;
R₁₇ is heterocyclyl, or alkyl optionally substituted by one or two groups selected from H₂N--, aryl-alkyl--, or alkyl-C(O)-NH--;
R₁₈ is heterocyclyl-alkyl--; or
a pharmaceutically acceptable salt thereof; or an optical isomer thereof; or a mixture of optical isomers.

The agent of the invention can be a [2,6] Naphtyhyridine according to Formula I wherein
R₁ and R₂ are independently hydrogen, (C₁-C₇)alkyl, (C₃-C₇)cycloalkyl, heterocyclyl, each of which is optionally substituted by one to two R₈, wherein R₈ is hydrogen, halogen, (C₁-C₇)alkyl, R₉-O--, (R₁₀)(R₁₁)N--, (R₁₂)(R₁₃)N-C(O)--, (C₆-C₁₀)aryl, or heterocyclyl or heteroaryl, said heterocyclyl and heteroaryl are optionally substituted by one or two (C₁-C₇)alkyl groups;
R₁ and R₂ taken together with the nitrogen atom to which they are attached to optionally form a 4-7 membered ring;
R₃ is (R₁₄)(R₁₅)N;
R₄, R₅, R₆ are H; and
R₇ is independently hydrogen, halogen, (C₁-C₇)alkyl, (C₃-C₇)cycloalkyl, (C₆-C₁₀)aryl-(C₁-C₇)alkyl, (C₆-C₁₀)aryl, or (C₁-C₇)alkoxy;
R₉, R₁₀, R₁₁, R₁₂ and R₁₃ are independently hydrogen, (C₁-C₇)alkyl-O-C(O)--, (C₁-C₇)alkyl-NH-C(O)--, (C₁-C₇)alkyl-C(O)-NH-C(O)--, (C₃-C₇)cycloalkyl, (C₃-C₇)cycloalkyl-(C₁-C₇)alkyl--, R₁₆-SO₂-- R₁₇-C(O)--, heterocyclyl or (C₁-C₇)alkyl, said heterocyclyl is further optionally substituted by one or two (C₃-C₇)cycloalkyl-(C₁-C₇)alkyl-- groups, and said alkyl is further optionally substituted by one or two groups selected from hydroxy, (C₁-C₇)alkoxy, (C₁-C₇)alkylamine, di-(C₁-C₇)alkylamine, or heteroaryl;
R₁₀ and R₁₁ taken together with the nitrogen atom to which they are attached to optionally form a 5-7 membered ring;
R₁₂ and R₁₃ taken together with the nitrogen atom to which they are attached to optionally form a 5-7 membered ring;
R₁₄ and R₁₅ are independently hydrogen, (C₁-C₇)alkyl, (C₆-C₁₀)aryl, (C₃-C₇)cycloalkyl, (C₆-C₁₀)aryl-(C₁-C₇)alkyl--, heterocyclyl or heteroaryl, said alkyl, cycloalkyl, aryl and heteroaryl are further optionally substituted by one or two groups selected from (C₁-C₇)alkyl, (C₁-C₇)alkoxy, hydroxy, halogen, halo(C₁-C₇)alkyl, cyano, or R₁₈-NH-C(O)--;
R₁₆ is aryl or heteroaryl;
R₁₇ is heterocyclyl, or (C₁-C₇)alkyl optionally substituted by one or two groups selected from H₂N--, (C₆-C₁₀)aryl-(C₁-C₇)alkyl--, or (C₁-C₇)alkyl-C(O)-NH--;
R₁₈ is heterocyclyl-(C₁-C₇)alkyl-; or
wherein heterocyclyl refers to an optionally substituted, saturated or unsaturated non-aromatic ring or ring system, which is a 4-, 5-, 6-, or 7-membered monocyclic; and
heteroaryl refers to a 5-14 membered monocyclic- or bicyclic- or polycyclic-aromatic ring system, having 1 to 8 heteroatoms selected from N, O or S;
a pharmaceutically acceptable salt thereof; or an optical isomer thereof; or a mixture of optical isomers

In some embodiments, the inhibitor is the cyclohexyl-[4-(4-phenyl-1-piperazin-1-yl-[2,6]naphthyridin-3-yl)pyridin-2-yl]amine of formula (II):

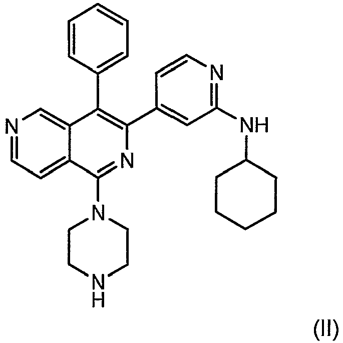

### Description of the figures

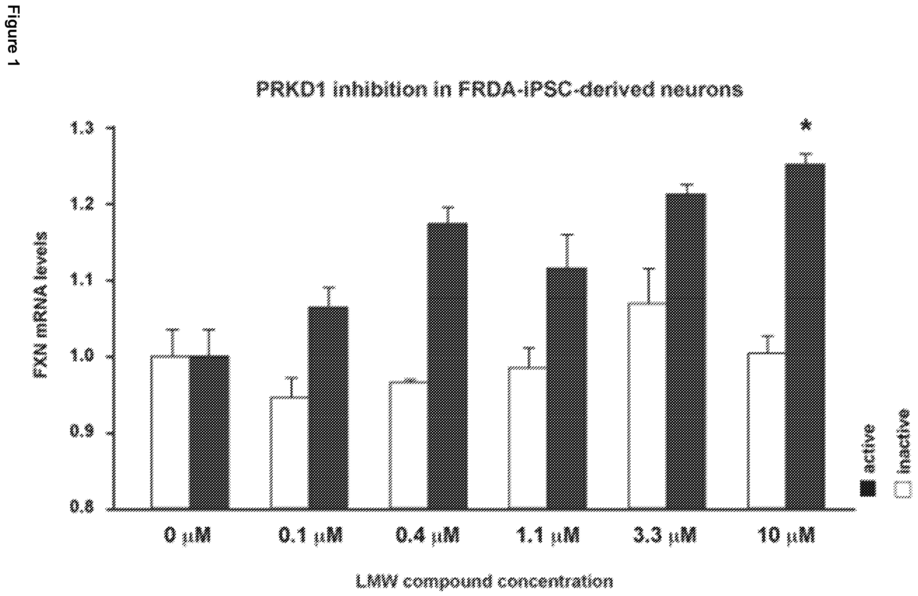

- Fig. 1:: **Exposure of FRDA-iPSC-derived neurons to [2,6] Naphtyhyridine is the cyclohexyl[4-(4-phenyl-1-piperazin-1-yl-[2,6]naphthyridin-3-yl)pyridin-2-yl]amine, a chemical inhibitor of PRKD1, leads to an increase in FXN expression while a control compound has no effect.** Neuron cultures were treated for 3 weeks with five different concentrations of the PRKD1 inhibitor (**active**), or an inactive analog (**inactive**) as described in the Examples. The experiment was run in biological duplicates and no compound toxicity was observed at the concentrations used. FXN mRNA levels were determined by real-time RT-PCR, normalized to RPL13A mRNA levels. **P* = 0.03 .

### Detailed description of the invention

In order to address the needs in the art, the present inventors generated a reporter cell line of human origin, characterized in that a reporter gene has been fused to the 3'-end of the endogenous frataxin (FTX) gene, wherein said reporter cell line is compatible with high-throughput biology and enables accurate monitoring of endogenous FXN gene expression. Using this cell line, the present inventors have been able to identify a new physiologically-relevant link between PKD and FRDA and showed for the first time that PKD inhibitors are agents which can be used to treat capable FRDA.

The present invention hence encompasses an agent which modulate the activity of the *PRKD1* gene product, protein kinase D1, for use in the treatment of Friedreich's ataxia as defined in the claims. In some embodiments, the protein kinase D1 inhibitor is a [2,6] Naphtyhyridine of formula (I): 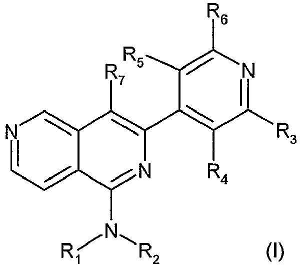 wherein
R₁ and R₂ are independently hydrogen, alkyl, cycloalkyl, heterocyclyl, each of which is optionally substituted by one to two R₈, wherein R₈ is hydrogen, halogen, alkyl, R₉-O--, (R₁₀)(R₁₁)N--, (R₁₂)(R₁₃)N-C(O)--, aryl, or heterocyclyl or heteroaryl, said heterocyclyl and heteroaryl are optionally substituted by one or two alkyl groups;
R₁ and R₂ taken together with the nitrogen atom to which they are attached to optionally form a 4-7 membered ring;
R₃ is (R₁₄)(R₁₅)N--, or halogen;
R₄, R₅, R₆ and R₇ are independently hydrogen, halogen, alkyl, (C₃-C₇) cycloalkyl, aryl-alkyl, aryl, or alkoxy;
R₉, R₁₀, R₁₁, R₁₂ and R₁₃ are independently hydrogen, alkyl-O-C(O)--, alkyl-NH-C(O)--, alkyl-C(O)-NH-C(O)--, cycloalkyl, cycloalkyl-alkyl--, R₁₆-SO₂-, R₁₇-C(O)--, heterocyclyl or alkyl, said heterocyclyl is further optionally substituted by one or two cycloalkyl-alkyl-- groups, and said alkyl is further optionally substituted by one or two groups selected from hydroxy, alkoxy, alkylamine, dialkylamine, or heteroaryl;
R₁₀ and R₁₁ taken together with the nitrogen atom to which they are attached to optionally form a 5-7 membered ring;
R₁₂ and R₁₃ taken together with the nitrogen atom to which they are attached to optionally form a 5-7 membered ring;
R₁₄ and R₁₅ are independently hydrogen, alkyl, aryl, cycloalkyl, aryl-alkyl--, heterocyclyl or heteroaryl, said alkyl, cycloalkyl, aryl and heteroaryl are further optionally substituted by one or two groups selected from alkyl, alkoxy, hydroxy, halogen, haloalkyl, cyano, or R₁₈-NH-C(O)--;
R₁₆ is aryl or heteroaryl;
R₁₇ is heterocyclyl, or alkyl optionally substituted by one or two groups selected from H₂N--, aryl-alkyl--, or alkyl-C(O)-NH--;
R₁₈ is heterocyclyl-alkyl--; or
a pharmaceutically acceptable salt thereof; or an optical isomer thereof; or a mixture of optical isomers.

The agent of the invention can be a [2,6] Naphtyhyridine according to Formula I wherein
R₁ and R₂ are independently hydrogen, (C₁-C₇)alkyl, (C₃-C₇)cycloalkyl, heterocyclyl, each of which is optionally substituted by one to two R₈, wherein R₈ is hydrogen, halogen, (C₁-C₇)alkyl, R₉-O--, (R₁₀)(R₁₁)N--, (R₁₂)(R₁₃)N-C(O)--, (C₆-C₁₀)aryl, or heterocyclyl or heteroaryl, said heterocyclyl and heteroaryl are optionally substituted by one or two (C₁-C₇)alkyl groups;
R₁ and R₂ taken together with the nitrogen atom to which they are attached to optionally form a 4-7 membered ring;
R₃ is (R₁₄)(R₁₅)N;
R₄, R₅, R₆ are H; and
R₇ is independently hydrogen, halogen, (C₁-C₇)alkyl, (C₃-C₇)cycloalkyl, (C₆-C₁₀)aryl-(C₁-C₇)alkyl, (C₆-C₁₀)aryl, or (C₁-C₇)alkoxy;
R₉, R₁₀, R₁₁, R₁₂ and R₁₃ are independently hydrogen, (C₁-C₇)alkyl-O-C(O)--, (C₁-C₇)alkyl-NH-C(O)--, (C₁-C₇)alkyl-C(O)-NH-C(O)--, (C₃-C₇)cycloalkyl, (C₃-C₇)cycloalkyl-(C₁-C₇)alkyl--, R₁₆-SO₂--, R₁₇-C(O)--, heterocyclyl or (C₁-C₇)alkyl, said heterocyclyl is further optionally substituted by one or two (C₃-C₇)cycloalkyl-(C₁-C₇)alkyl-- groups, and said alkyl is further optionally substituted by one or two groups selected from hydroxy, (C₁-C₇)alkoxy, (C₁-C₇)alkylamine, di-(C₁-C₇)alkylamine, or heteroaryl;
R₁₀ and R₁₁ taken together with the nitrogen atom to which they are attached to optionally form a 5-7 membered ring;
R₁₂ and R₁₃ taken together with the nitrogen atom to which they are attached to optionally form a 5-7 membered ring;
R₁₄ and R₁₅ are independently hydrogen, (C₁-C₇)alkyl, (C₆-C₁₀)aryl, (C₃-C₇)cycloalkyl, (C₆-C₁₀)aryl-(C₁-C₇)alkyl--, heterocyclyl or heteroaryl, said alkyl, cycloalkyl, aryl and heteroaryl are further optionally substituted by one or two groups selected from (C₁-C₇)alkyl, (C₁-C₇)alkoxy, hydroxy, halogen, halo(C₁-C₇)alkyl, cyano, or R₁₈-NH-C(O)--;
R₁₆ is aryl or heteroaryl;
R₁₇ is heterocyclyl, or (C₁-C₇)alkyl optionally substituted by one or two groups selected from H₂N--, (C₆-C₁₀)aryl-(C₁-C₇)alkyl--, or (C₁-C₇)alkyl-C(O)-NH--;
R₁₈ is heterocyclyl-(C₁-C₇)alkyl--; or
wherein heterocyclyl refers to an optionally substituted, saturated or unsaturated non-aromatic ring or ring system, which is a 4-, 5-, 6-, or 7-membered monocyclic; and
heteroaryl refers to a 5-14 membered monocyclic- or bicyclic- or polycyclic-aromatic ring system, having 1 to 8 heteroatoms selected from N, O or S;
a pharmaceutically acceptable salt thereof; or an optical isomer thereof; or a mixture of optical isomers

In some embodiments, the inhibitor is the cyclohexyl-[4-(4-phenyl-1-piperazin-1-yl-[2,6]naphthyridin-3-yl)pyridin-2-yl]amine of formula (II):

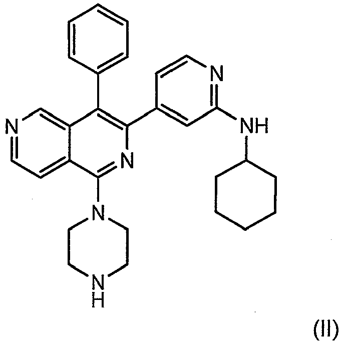

For purposes of interpreting this specification, the following definitions will apply and whenever appropriate, terms used in the singular will also include the plural and vice versa.

As used herein, the term "alkyl" refers to a fully saturated branched or unbranched hydrocarbon moiety. In some embodiments the alkyl comprises 1 to 20 carbon atoms. In some embodiments 1 to 16 carbon atoms, 1 to 10 carbon atoms, 1 to 7 carbon atoms, or 1 to 4 carbon atoms. In some embodiments, (C1-C7) refer to an alkyl containing 1 to 7 carbon atoms. Representative examples of alkyl include, but are not limited to, methyl, ethyl, *n*-propyl, *iso*-propyl, *n*-butyl, *sec*-butyl, *iso*-butyl, *tert-*butyl, *n*-pentyl, isopentyl, neopentyl, *n*-hexyl, 3-methylhexyl, 2,2- dimethylpentyl, 2,3-dimethylpentyl, *n-*heptyl, *n*-octyl, *n*-nonyl, *n*-decyl and the like.

As used herein, the term "haloalkyl" refers to an alkyl as defined herein, that is substituted by one or more halo groups as defined herein. The haloalkyl can be monohaloalkyl, dihaloalkyl or polyhaloalkyl including perhaloalkyl. A monohaloalkyl can have one iodo, bromo, chloro or fluoro within the alkyl group. Dihaloalky and polyhaloalkyl groups can have two or more of the same halo atoms or a combination of different halo groups within the alkyl. The polyhaloalkyl contains up to 12, or 10, or 8, or 6, or 4, or 3, or 2 halo groups. Non-limiting examples of haloalkyl include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. A perhaloalkyl refers to an alkyl having all hydrogen atoms replaced with halo atoms.

The term "aryl" refers to monocyclic or bicyclic aromatic hydrocarbon groups having 6-20 carbon atoms in the ring portion. In some embodiments, the aryl is a (C₆-C₁₀) aryl. Non-limiting examples include phenyl, naphthyl or tetrahydronaphthyl.

Furthermore, the term "aryl" as used herein, refers to an aromatic substituent which can be a single aromatic ring, or multiple aromatic rings that are fused together. The common linking group also can be a carbonyl as in benzophenone or oxygen as in diphenylether or nitrogen as in diphenylamine.

As used herein, the term "alkoxy" refers to alkyl-O-, wherein alkyl is defined herein above. Representative examples of alkoxy include, but are not limited to, methoxy, ethoxy, propoxy, 2-propoxy, butoxy, *tert*-butoxy, pentyloxy, hexyloxy, cyclopropyloxy-, cyclohexyloxy- and the like. In some embodiments, alkoxy groups have 1-7. In some embodiments, they have 1-4 carbons.

As used herein, the term "acyl" refers to a group R-C(O)- of from 1 to 10 carbon atoms of a straight, branched, or cyclic configuration or a combination thereof, attached to the parent structure through carbonyl functionality. Such group can be saturated or unsaturated, and aliphatic or aromatic. In some embodiments, R in the acyl residue is alkyl, or alkoxy, or aryl, or heteroaryl. Also In some embodiments, one or more carbons in the acyl residue may be replaced by nitrogen, oxygen or sulfur as long as the point of attachment to the parent remains at the carbonyl. Examples of acyl include but are not limited to, acetyl, benzoyl, propionyl, isobutyryl, t- butoxycarbonyl, benzyloxycarbonyl and the like. Lower acyl refers to acyl containing one to four carbons.

As used herein, the term "heterocyclyl" or "heterocyclo" refers to an optionally substituted, saturated or unsaturated non-aromatic ring or ring system, e.g., which is a 4-, 5-, 6-, or 7-membered monocyclic, 7-, 8-, 9-, 10-, 11-, or 12-membered bicyclic or 10-, 11-, 12-, 13-, 14- or 15-membered tricyclic ring system and contains at least one heteroatom selected from O, S and N, where the N and S can also optionally be oxidized to various oxidation states. The heterocyclic group can be attached at a heteroatom or a carbon atom. The heterocyclyl can include fused or bridged rings as well as spirocyclic rings. Examples of heterocycles include tetrahydrofuran (THF), dihydrofurari, 1, 4-dioxane, morpholine, 1,4-dithiane, piperazine, piperidine, 1,3-dioxolane, imidazolidine, imidazoline, pyrroline, pyrrolidine, tetrahydropyran, dihydropyran, oxathiolane, dithiolane, l,3-dioxane, 1,3-dithiane, oxathiane, thiomorpholine, and the like.

As used herein, the term "cycloalkyl" refers to saturated or unsaturated monocyclic, bicyclic or tricyclic hydrocarbon groups of 3-12 carbon atoms, in some embodiments 3-9, or 3-7 carbon atoms. Exemplary monocyclic hydrocarbon groups include, but are not limited to, cyclopropyl, cyclobutyl, cyclopentyl, cyclopentenyl, cyclohexyl and cyclohexenyl and the like. Exemplary bicyclic hydrocarbon groups include bornyl, indyl, hexahydroindyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl and the like. Exemplary tricyclic hydrocarbon groups include adamantyl and the like.

As used herein, the term "heteroaryl" refers to a 5-14 membered monocyclic- or bicyclic- or polycyclic-aromatic ring system, having 1 to 8 heteroatoms selected from N, O or S. In some embodiments, the heteroaryl is a 5-10 or 5-7 membered ring system. Typical heteroaryl groups include 2- or 3-thienyl, 2- or 3-furyl, 2- or 3-pyrrolyl, 2-, 4-, or 5-imidazolyl, 3-, 4-, or 5- pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2, 3-triazolyl, tetrazolyl, 2-, 3-, or 4-pyridyl, 3- or 4-pyridazinyl, 3-, 4-, or 5-pyrazinyl, 2-pyrazinyl, 2-, 4-, or 5-pyrimidinyl.

The term "heteroaryl" also refers to a group in which a heteroaromatic ring is fused to one or more aryl, cycloaliphatic, or heterocyclyl rings, where the radical or point of attachment is on the heteroaromatic ring. Nonlimiting examples include but are not limited to 1-, 2-, 3-, 5-, 6-, 7-, or 8-indolizinyl, 1-, 3-, 4-, 5-, 6-, or 7-isoindolyl, 2-, 3-, 4-, 5-, 6-, or 7-indolyl, 2-, 3-, 4-, 5-, 6-, or 7-indazolyl, 2-, 4-, 5-, 6-, 7-, or 8- purinyl, 1-, 2-, 3-, 4-, 6-, 7-, 8-, or 9-quinolizinyl, 2-, 3-, 4-, 5-, 6-, 7-, or 8-quinoliyl, 1-, 3-, 4-, 5-, 6-, 7-, or 8-isoquinoliyl, 1-, 4-, 5-, 6-, 7-, or 8-phthalazinyl, 2-, 3-, 4-, 5-, or 6-naphthyridinyl, 2-, 3-, 5-, 6-, 7-, or 8-quinazolinyl, 3-, 4-, 5-, 6-, 7-, or 8-cinnolinyl, 2-, 4-, 6-, or 7-pteridinyl, 1-, 2-, 3-, 4-, 5-, 6-, 7-, or 8-4aH carbazolyl, 1-, 2-, 3-, 4-, 5-, 6-, 7-, or 8-carbzaolyl, 1-, 3-, 4-, 5-, 6-, 7-, 8-, or 9-carbolinyl, 1-, 2-, 3-, 4-, 6-, 7-, 8-, 9-, or 10-phenanthridinyl, 1-, 2-, 3-, 4-, 5-, 6-, 7-, 8-, or 9-acridinyl, 1-, 2-, 4-, 5-, 6-, 7-, 8-, or 9-perimidinyl, 2-, 3-, 4-, 5-, 6-, 8-, 9-, or 10-phenathrolinyl, 1-, 2-, 3-, 4-, 6-, 7-, 8-, or 9-phenazinyl, 1-, 2-, 3-, 4-, 6-, 7-, 8-, 9-, or 10-phenothiazinyl, 1-, 2-, 3-, 4-, 6-, 7-, 8-, 9-, or 10-phenoxazinyl, 2-, 3-, 4-, 5-, 6-, or 2-, 3-, 4-, 5-, 6-, 7-, 8-, 9-, or 10- benzisoqinolinyl, 2-, 3-, 4-, or thieno[2,3-b]furanyl, 2-, 3-, 5-, 6-, 7-, 8-, 9-, 10 -, or 11-7H-pyrazino[2,3-c]carbazolyl,2-, 3-, 5-, 6-, or 7-2H- furo[3,2-b]-pyranyl, 2-, 3-, 4-, 5-, 7-, or 8-5H-pyrido[2,3-d]-o-oxazinyl, 1-, 3-, or 5-1H-pyrazolo[4,3-d]-oxazolyl, 2-, 4-, or 54H-imidazo[4,5-d] thiazolyl, 3-, 5-, or 8-pyrazino[2,3-d]pyridazinyl, 2-, 3-, 5-, or 6- imidazo[2,1-b] thiazolyl, 1-, 3-, 6-, 7-, 8-, or 9-furo[3,4-c]cinnolinyl, 1-, 2-, 3-, 4-, 5-, 6-, 8-, 9-, 10, or 11-4H-pyrido[2,3-c]carbazolyl, 2-, 3-, 6-, or 7-imidazo[1,2-b][1,2,4]triazinyl, 7-benzo[b]thienyl, 2-, 4-, 5- , 6-, or 7-benzoxazolyl, 2-, 4-, 5-, 6-, or 7-benzimidazolyl, 2-, 4-, 4-, 5-, 6-, or 7-benzothiazolyl, 1-, 2-, 4-, 5-, 6-, 7-, 8-, or 9- benzoxapinyl, 2-, 4-, 5-, 6-, 7-, or 8-benzoxazinyl, 1-, 2-, 3-, 5-, 6-, 7-, 8-, 9-, 10-, or 11-1H-pyrrolo[1,2-b][2]benzazapinyl. Typical fused heteroary groups include, but are not limited to 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, 2-, 4-, 5-, 6-, or 7-benzoxazolyl, 2-, 4-, 5-, 6-, or 7-benzimidazolyl, 2-, 4-, 5-, 6-, or 7-benzothiazolyl. A heteroaryl group may be mono-, bi-, tri-, or polycyclic. In some embodiments it may be mono-, bi-, or tricyclic. In some embodiments it may be mono- or bicyclic.

As used herein, the term "halogen" or "halo" refers to fluoro, chloro, bromo, and iodo.

As used herein, the term "isomers" refers to different compounds that have the same molecular formula but differ in arrangement and configuration of the atoms. Also as used herein, the term "an optical isomer" or "a stereoisomer" refers to any of the various stereo isomeric configurations which may exist for a given compound of the present invention and includes geometric isomers. It is understood that a substituent may be attached at a chiral center of a carbon atom. Therefore, the invention includes enantiomers, diastereomers or racemates of the compound. "Enantiomers" are a pair of stereoisomers that are non- superimposable mirror images of each other. A 1:1 mixture of a pair of enantiomers is a "racemic" mixture. The term is used to designate a racemic mixture where appropriate. "Diastereoisomers" are stereoisomers that have at least two asymmetric atoms, but which are not mirror-images of each other. The absolute stereochemistry is specified according to the Cahn-Ingold- Prelog R-S system. When a compound is a pure enantiomer the stereochemistry at each chiral carbon may be specified by either *R* or *S*. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers and may thus give rise to enantiomers, diastereomers, and other stereoisomeric forms that may be defined, in terms of absolute stereochemistry, as (*R*)- or (*S*)-. The present invention is meant to include all such possible isomers, including racemic mixtures, optically pure forms and intermediate mixtures. Optically active (*R*)- and (*S*)- isomers may be prepared using chiral synthons or chiral reagents, or resolved using conventional techniques. If the compound contains a double bond, the substituent may be E or Z configuration. If the compound contains a disubstituted cycloalkyl, the cycloalkyl substituent may have a cis- or trans-configuration. All tautomeric forms are also intended to be included.

As used herein, the term "pharmaceutically acceptable salts" refers to salts that retain the biological effectiveness and properties of the compounds of this invention and, which are not biologically or otherwise undesirable. In many cases, the compounds of the present invention are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, *p*- toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like; particularly preferred are the ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. The pharmaceutically acceptable salts of the present invention can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, non-aqueous media like ether, ethyl acetate, ethanol, isopropanol, or acetonitrile are preferred, where practicable. Lists of additional suitable salts can be found, *e*.*g*., in Remington's Pharmaceutical Sciences, 20th ed., Mack Publishing Company, Easton, Pa., (1985).

As used herein, the term "pharmaceutically acceptable carrier" includes any and all solvents, dispersion media, coatings, surfactants, antioxidants, preservatives (*e*.*g*., antibacterial agents, antifungal agents), isotonic agents, absorption delaying agents, salts, preservatives, drugs, drug stabilizers, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.

The term "a therapeutically effective amount" of a compound of the present invention refers to an amount of the compound of the present invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the compound of the present invention that, when administered to a subject, is effective to (1) at least partially alleviating, inhibiting, preventing and/or ameliorating Friedreich' ataxia.

As used herein, the term "subject" refers to an animal. In some embodiments, the animal is a mammal. A subject also refers to for example, primates (*e*.*g*., humans), cows, sheep, goats, horses, camels, dogs, cats, rabbits, rats, mice, fish, birds and the like.

As used herein, the term "a disorder" or "a disease" refers to any derangement or abnormality of function; a morbid physical or mental state. See Dorland's Illustrated Medical Dictionary, (W.B. Saunders Co. 27th ed. 1988).

As used herein, the term "treating" or "treatment" of any disease or disorder refers in one embodiment, to ameliorating the disease or disorder (i.e., slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (*e*.*g*., stabilization of a discernible symptom), physiologically, (*e*.*g*., stabilization of a physical parameter), or both. In yet another embodiment, "treating" or "treatment" refers to preventing or delaying the onset or development or progression of the disease or disorder.

As used herein, the term "abnormal" refers to an activity or feature which differs from a normal activity or feature.

As used herein, the term "abnormal activity" refers to an activity which differs from the activity of the wild-type or native gene or protein, or which differs from the activity of the gene or protein in a healthy subject. The abnormal activity can be stronger or weaker than the normal activity. In one embodiment, the "abnormal activity" includes the abnormal (either over- or under-) production of mRNA transcribed from a gene. In another embodiment, the "abnormal activity" includes the abnormal (either over- or under-) production of polypeptide from a gene. In another embodiment, the abnormal activity refers to a level of a mRNA or polypeptide that is different from a normal level of said mRNA or polypeptide by about 15%, about 25%, about 35%, about 50%, about 65%, about 85%, about 100% or greater. In some embodiments, the abnormal level of the mRNA or polypeptide can be either higher or lower than the normal level of said mRNA or polypeptide. Yet in another embodiment, the abnormal activity refers to functional activity of a protein that is different from a normal activity of the wild-type protein. In some embodiments, the abnormal activity can be stronger or weaker than the normal activity. In some embodiments, the abnormal activity is due to the mutations in the corresponding gene, and the mutations can be in the coding region of the gene or non-coding regions such as transcriptional promoter regions. The mutations can be substitutions, deletions, insertions.

As used herein, the term "a," "an," "the" and similar terms used in the context of the present invention (especially in the context of the claims) are to be construed to cover both the singular and plural unless otherwise indicated herein or clearly contradicted by the context. Recitation of ranges of values herein are merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (*e*.*g*. "such as") provided herein is intended merely to better illuminate the invention and does not pose a limitation on the scope of the invention otherwise claimed. No language in the specification should be construed as indicating any non-claimed element essential to the practice of the invention.

Any asymmetric carbon atom on the compounds of the present invention can be present in the (R)-, (*S*)- or (*R*,*S*)- configuration, In some embodiments in the (*R*)- or (*S*)- configuration. Substituents at atoms with unsaturated bonds may, if possible, be present in *cis-* (*Z*)- or *trans-* (*E*)- form. Therefore, the compounds of the present invention can be in the form of one of the possible isomers or mixtures thereof, for example, as substantially pure geometric (*cis* or *trans*) isomers, diastereomers, optical isomers (antipodes), racemates or mixtures thereof. Any resulting mixtures of isomers can be separated on the basis of the physicochemical differences of the constituents, into the pure geometric or optical isomers, diastereomers, racemates, for example, by chromatography and/or fractional crystallization. Any resulting racemates of final products or intermediates can be resolved into the optical antipodes by known methods, *e*.*g*., by separation of the diastereomeric salts thereof, obtained with an optically active acid or base, and liberating the optically active acidic or basic compound. In particular, the imidazolyl moiety may thus be employed to resolve the compounds of the present invention into their optical antipodes, *e*.*g*., by fractional crystallization of a salt formed with an optically active acid, *e*.*g*., tartaric acid, dibenzoyl tartaric acid, diacetyl tartaric acid, di-*O*,*O'*-*p*-toluoyl tartaric acid, mandelic acid, malic acid or camphor-10-sulfonic acid. Racemic products can also be resolved by chiral chromatography, *e*.*g*., high pressure liquid chromatography (HPLC) using a chiral adsorbent. Finally, compounds of the present invention are either obtained in the free form, as a salt thereof, or as prodrug derivatives thereof.

When a basic group is present in the compounds of the present invention, the compounds can be converted into acid addition salts thereof, in particular, acid addition salts with the imidazolyl moiety of the structure, In some embodiments pharmaceutically acceptable salts thereof. These are formed, with inorganic acids or organic acids. Suitable inorganic acids include but are not limited to, hydrochloric acid, sulfuric acid, a phosphoric or hydrohalic acid. Suitable organic acids include but are not limited to, carboxylic acids, such as (C₁-C₄)alkanecarboxylic acids which, for example, are unsubstituted or substituted by halogen, *e*.*g*., acetic acid, such as saturated or unsaturated dicarboxylic acids, *e*.*g*., oxalic, succinic, maleic or fumaric acid, such as hydroxycarboxylic acids, *e*.*g*., glycolic, lactic, malic, tartaric or citric acid, such as amino acids, *e*.*g*., aspartic or glutamic acid, organic sulfonic acids, such as (C₁-C₄)alkylsulfonic acids, *e*.*g*., methanesulfonic acid; or arylsulfonic acids which are unsubstituted or substituted, *e*.*g*., by halogen. Preferred are salts formed with hydrochloric acid, methanesulfonic acid and maleic acid.

When an acidic group is present in the compounds of the present invention, the compounds can be converted into salts with pharmaceutically acceptable bases. Such salts include alkali metal salts, like sodium, lithium and potassium salts; alkaline earth metal salts, like calcium and magnesium salts; ammonium salts with organic bases, *e*.*g*., trimethylamine salts, diethylamine salts, *tris*(hydroxymethyl)methylamine salts, dicyclohexylamine salts and *N*-methyl-*D*-glucamine salts; salts with amino acids like arginine, lysine and the like. Salts may be formed using conventional methods, advantageously in the presence of an ethereal or alcoholic solvent, such as a lower alkanol. From the solutions of the latter, the salts may be precipitated with ethers, *e*.*g*., diethyl ether. Resulting salts may be converted into the free compounds by treatment with acids. These or other salts can also be used for purification of the compounds obtained.

When both a basic group and an acid group are present in the same molecule, the compounds of the present invention can also form internal salts.

The present invention also provides pro-drugs of the compounds of the present invention that converts *in vivo* to the compounds of the present invention. A pro-drug is an active or inactive compound that is modified chemically through *in vivo* physiological action, such as hydrolysis, metabolism and the like, into a compound of this invention following administration of the prodrug to a subject. The suitability and techniques involved in making and using pro-drugs are well known by those skilled in the art. Prodrugs can be conceptually divided into two non-exclusive categories, bioprecursor prodrugs and carrier prodrugs. See The Practice of Medicinal Chemistry, Ch. 31-32 (Ed. Wermuth, Academic Press, San Diego, Calif., 2001). Generally, bioprecursor prodrugs are compounds are inactive or have low activity compared to the corresponding active drug compound, that contains one or more protective groups and are converted to an active form by metabolism or solvolysis. Both the active drug form and any released metabolic products should have acceptably low toxicity. Typically, the formation of active drug compound involves a metabolic process or reaction that is one of the follow types:
1. Oxidative reactions, such as oxidation of alcohol, carbonyl, and acid functions, hydroxylation of aliphatic carbons, hydroxylation of alicyclic carbon atoms, oxidation of aromatic carbon atoms, oxidation of carbon-carbon double bonds, oxidation of nitrogen-containing functional groups, oxidation of silicon, phosphorus, arsenic, and sulfur, oxidative N-delakylation, oxidative O- and S-dealkylation, oxidative deamination, as well as other oxidative reactions.
2. Reductive reactions, such as reduction of carbonyl groups, reduction of alcoholic groups and carbon-carbon double bonds, reduction of nitrogen-containing functions groups, and other reduction reactions.
3. Reactions without change in the state of oxidation, such as hydrolysis of esters and ethers, hydrolytic cleavage of carbon-nitrogen single bonds, hydrolytic cleavage of non-aromatic heterocycles, hydration and dehydration at multiple bonds, new atomic linkages resulting from dehydration reactions, hydrolytic dehalogenation, removal of hydrogen halide molecule, and other such reactions. Carrier prodrugs are drug compounds that contain a transport moiety, *e*.*g*., that improve uptake and/or localized delivery to a site(s) of action. Desirably for such a carrier prodrug, the linkage between the drug moiety and the transport moiety is a covalent bond, the prodrug is inactive or less active than the drug compound, and any released transport moiety is acceptably non-toxic. For prodrugs where the transport moiety is intended to enhance uptake, typically the release of the transport moiety should be rapid. In other cases, it is desirable to utilize a moiety that provides slow release, *e*.*g*., certain polymers or other moieties, such as cyclodextrins. See, Cheng et al., US20040077595, application Ser. No. 10/656,838. Such carrier prodrugs are often advantageous for orally administered drugs. Carrier prodrugs can, for example, be used to improve one or more of the following properties: increased lipophilicity, increased duration of pharmacological effects, increased site-specificity, decreased toxicity and adverse reactions, and/or improvement in drug formulation (*e*.*g*., stability, water solubility, suppression of an undesirable organoleptic or physiochemical property). For example, lipophilicity can be increased by esterification of hydroxyl groups with lipophilic carboxylic acids, or of carboxylic acid groups with alcohols, *e*.*g*., aliphatic alcohols. Wermuth, The Practice of Medicinal Chemistry, Ch. 31-32, Ed. Werriuth, Academic Press, San Diego, Calif., 2001.

Exemplary prodrugs are, *e*.*g*., esters of free carboxylic acids and *S*-acyl and *O*-acyl derivatives of thiols, alcohols or phenols, wherein acyl has a meaning as defined herein. Preferred are pharmaceutically acceptable ester derivatives convertible by solvolysis under physiological conditions to the parent carboxylic acid, *e*.*g*., lower alkyl esters, cycloalkyl esters, lower alkenyl esters, benzyl esters, mono- or di-substituted lower alkyl esters, such as the ω-(amino, mono- or di-lower alkylamino, carboxy, lower alkoxycarbonyl)-lower alkyl esters, the α-(lower alkanoyloxy, lower alkoxycarbonyl or di-lower alkylaminocarbonyl)-lower alkyl esters, such as the pivaloyloxymethyl ester and the like conventionally used in the art. In addition, amines have been masked as arylcarbonyloxymethyl substituted derivatives which are cleaved by esterases *in vivo* releasing the free drug and formaldehyde (Bundgaard, J. Med. Chem. 2503 (1989)). Moreover, drugs containing an acidic NH group, such as imidazole, imide, indole and the like, have been masked with N-acyloxymethyl groups (Bundgaard, Design of Prodrugs, Elsevier (1985)). Hydroxy groups have been masked as esters and ethers. EP 039,051 (Sloan and Little) discloses Mannich-base hydroxamic acid prodrugs, their preparation and use.

In view of the close relationship between the compounds, the compounds in the form of their salts and the pro-drugs, any reference to the compounds of the present invention is to be understood as referring also to the corresponding pro-drugs of the compounds of the present invention, as appropriate and expedient.

Furthermore, the compounds of the present invention, including their salts, can also be obtained in the form of their hydrates, or include other solvents used for their crystallization.

As used herein, the term "population" may be any group of at least two individuals. A population may include, *e*.*g*., but is not limited to, a reference population, a population group, a family population, a clinical population, and a same sex population.

As used herein, the term "polymorphism" means any sequence variant present at a frequency of >1% in a population. The sequence variant may be present at a frequency significantly greater than 1% such as 5% or 10% or more. Also, the term may be used to refer to the sequence variation observed in an individual at a polymorphic site. Polymorphisms include nucleotide substitutions, insertions, deletions and microsatellites and may, but need not, result in detectable differences in gene expression or protein function.

As used herein, the term "polynucleotide" means any RNA or DNA, which may be unmodified or modified RNA or DNA. Polynucleotides include, without limitation, single- and double-stranded DNA, DNA that is a mixture of single- and double-stranded regions, single- and double-stranded RNA, RNA that is mixture of single- and double-stranded regions, and hybrid molecules comprising DNA and RNA that may be single-stranded or, more typically, double-stranded or a mixture of single- and double-stranded regions. In addition, polynucleotide refers to triple-stranded regions comprising RNA or DNA or both RNA and DNA. The term polynucleotide also includes DNAs or RNAs containing one or more modified bases and DNAs or RNAs with backbones modified e.g. for stability or for other reasons.

As used herein, the term "polypeptide" means any polypeptide comprising two or more amino acids joined to each other by peptide bonds or modified peptide bonds, *i*.*e*., peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well-known in the art. Such modifications are well described in basic texts and in more detailed monographs, as well as in a voluminous research literature.

As used herein, the term "reference standard population" means a population characterized by one or more biological characteristics, *e*.*g*., drug responsiveness, genotype, haplotype, phenotype, *etc*. As used herein, the term "subject" means that preferably the subject is a mammal, such as a human, but can also be an animal, including but not limited to, domestic animals (*e*.*g*., dogs, cats and the like), farm animals (*e*.*g*., cows, sheep, pigs, horses and the like) and laboratory animals (*e*.*g*., monkeys such as cynmologous monkeys, rats, mice, guinea pigs and the like).

As used herein, a "test sample" means a biological sample obtained from a subject of interest. For example, a test sample can be a biological fluid (*e*.*g*., serum), cell sample, or tissue, or isolated nucleic acid or polypeptide derived therefrom.

As used herein, the expression "body fluid" is a biological fluid selected from a group comprising blood, bile, blood plasma, serum, aqueous humor, amniotic fluid, cerebrospinal fluid, sebum, intestinal juice, semen, sputum, sweat and urine.

As used herein, the term "dysregulation" means a change that is larger or equal to 1.2 fold and statistically significant (p<0.05, Student's t-test) from the control. For example, a 1.5, 2, 2.5, 3, 3.5, 4, 4.5, or 5 fold change.

As used herein, the term "statistically significant" means a p value <0.05 as compared to the control using the Student's t-test.

The phrase "hybridising specifically to" as used herein refers to the binding, duplexing, or hybridising of an oligonucleotide probe preferentially to a particular target nucleotide sequence under stringent conditions when that sequence is present in a complex mixture (such as total cellular DNA or RNA). Preferably a probe may bind, duplex or hybridise only to the particular target molecule.

The term "stringent conditions" refers to conditions under which a probe will hybridise to its target subsequence, but minimally to other sequences. Preferably a probe may hybridise to no sequences other than its target under stringent conditions. Stringent conditions are sequence-dependent and will be different in different circumstances. Longer sequences hybridise specifically at higher temperatures. In general, stringent conditions may be selected to be about 5°C lower than the thermal melting point (Tm) for the specific sequence at a defined ionic strength and pH. The Tm is the temperature (under defined ionic strength, pH, and nucleic acid concentration) at which 50% of the oligonucleotide probes complementary to a target nucleic acid hybridise to the target nucleic acid at equilibrium. As the target nucleic acids will generally be present in excess, at Tm, 50% of the probes are occupied at equilibrium. By way of example, stringent conditions will be those in which the salt concentration is at least about 0.01 to 1.0 M Na⁺ ion concentration (or other salts) at pH 7.0 to 8.3 and the temperature is at least about 30° C for short probes (e.g., 10 to 50 nucleotides). Stringent conditions may also be achieved with the addition of destabilizing agents such as formamide. Oligonucleotide probes may be used to detect complementary nucleic acid sequences (i.e., nucleic acid targets) in a suitable representative sample. Such complementary binding forms the basis of most techniques in which oligonucleotides may be used to detect, and thereby allow comparison of, expression of particular genes. Preferred technologies permit the parallel quantitation of the expression of multiple genes and include technologies where amplification and quantitation of species are coupled in real-time, such as the quantitative reverse transcription PCR technologies and technologies where quantitation of amplified species occurs subsequent to amplification, such as array technologies. Array technologies involve the hybridisation of samples, representative of gene expression within the subject or control sample, with a plurality of oligonucleotide probes wherein each probe preferentially hybridises to a disclosed gene or genes. Array technologies provide for the unique identification of specific oligonucleotide sequences, for example by their physical position (e.g., a grid in a two-dimensional array as commercially provided by Affymetrix Inc.) or by association with another feature (e.g. labelled beads as commercially provided by Illumina Inc or Luminex Inc). Oligonuleotide arrays may be synthesised *in situ* (e.g by light directed synthesis as commercially provided by Affymetrix Inc) or pre-formed and spotted by contact or ink-jet technology (as commercially provided by Agilent or Applied Biosystems). It will be apparent to those skilled in the art that whole or partial cDNA sequences may also serve as probes for array technology (as commercially provided by Clontech). Oligonucleotide probes may be used in blotting techniques, such as Southern blotting or northern blotting, to detect and compare gene expression (for example by means of cDNA or mRNA target molecules representative of gene expression). Techniques and reagents suitable for use in Southern or northern blotting techniques will be well known to those of skill in the art. Briefly, samples comprising DNA (in the case of Southern blotting) or RNA (in the case of northern blotting) target molecules are separated according to their ability to penetrate a gel of a material such as acrylamide or agarose. Penetration of the gel may be driven by capillary action or by the activity of an electrical field. Once separation of the target molecules has been achieved these molecules are transferred to a thin membrane (typically nylon or nitrocellulose) before being immobilized on the membrane (for example by baking or by ultraviolet radiation). Gene expression may then be detected and compared by hybridisation of oligonucleotide probes to the target molecules bound to the membrane.

In certain circumstances the use of traditional hybridisation protocols for comparing gene expression may prove problematic. For example blotting techniques may have difficulty distinguishing between two or more gene products of approximately the same molecular weight since such similarly sized products are difficult to separate using gels. Accordingly, in such circumstances it may be preferred to compare gene expression using alternative techniques, such as those described below.

Gene expression in a sample representing gene expression in a subject may be assessed with reference to global transcript levels within suitable nucleic acid samples by means of high-density oligonucleotide array technology. Such technologies make use of arrays in which oligonucleotide probes are tethered, for example by covalent attachment, to a solid support. These arrays of oligonucleotide probes immobilized on solid supports represent preferred components to be used in the methods and kits of the invention for the comparison of gene expression. Large numbers of such probes may be attached in this manner to provide arrays suitable for the comparison of expression of large numbers of genes. Other suitable methodologies that may be used in the comparison of nucleic acid targets representative of gene expression include, but are not limited to, nucleic acid sequence based amplification (NASBA); or rolling circle DNA amplification (RCA).

The term "epitopes," as used herein, refers to portions of a polypeptide having antigenic or immunogenic activity in an animal, in some embodiments, a mammal,for instance in a human. In an embodiment, the present invention encompasses a polypeptide comprising an epitope, as well as the polynucleotide encoding this polypeptide. An "immunogenic epitope," as used herein, is defined as a portion of a protein that elicits an antibody response in an animal, as determined by any method known in the art, for example, by the methods for generating antibodies described infra. (See, for example, Geysen et al., Proc. Natl. Acad. Sci. USA 81:3998-4002 (1983)). The term "antigenic epitope," as used herein, is defined as a portion of a protein to which an antibody can immuno specifically bind its antigen as determined by any method well known in the art, for example, by the immunoassays described herein. Immunospecific binding excludes non-specific binding but does not necessarily exclude cross-reactivity with other antigens. Antigenic epitopes need not necessarily be immunogenic. Fragments which function as epitopes may be produced by any conventional means. (See, e.g., Houghten, Proc. Natl. Acad. Sci. USA 82:5131-5135 (1985), further described in U.S. Patent No. 4,631,211).

As one of skill in the art will appreciate, and as discussed above, polypeptides comprising an immunogenic or antigenic epitope can be fused to other polypeptide sequences. For example, polypeptides may be fused with the constant domain of immunoglobulins (IgA, IgE, IgG, IgM), or portions thereof (CHI, CH2, CH3, or any combination thereof and portions thereof), or albumin (including but not limited to recombinant albumin (see, e.g., U.S. Patent No. 5,876, 969, issued March 2, 1999, EP Patent 0 413 622, and U.S. Patent No. 5,766,883, issued June 16, 1998)), resulting in chimeric polypeptides. Such fusion proteins may facilitate purification and may increase half-life *in vivo.* This has been shown for chimeric proteins consisting of the first two domains of the human CD4-polypeptide and various domains of the constant regions of the heavy or light chains of mammalian immunoglobulins. See, e.g., EP 394,827; Traunecker et al., Nature, 331:84-86 (1988).

Enhanced delivery of an antigen across the epithelial barrier to the immune system has been demonstrated for antigens (e.g., insulin) conjugated to an FcRn binding partner such as IgG or Fc fragments (see, e. g., PCT Publications WO 96/22024 and WO 99/04813). IgG Fusion proteins that have a disulfide-linked dimeric structure due to the IgG portion disulfide bonds have also been found to be more efficient in binding and neutralizing other molecules than monomeric polypeptides or fragments thereof alone. See, e.g., Fountoulakis et al., J. Blochem., 270:3958-3964 (1995). Nucleic acids encoding the above epitopes can also be recombined with a gene of interest as an epitope tag (e.g., the hemagglutinin ("HA") tag or flag tag) to aid in detection and punification of the expressed polypeptide. For example, a system described by Janknecht et al. allows for the ready purification of non-denatured fusion proteins expressed in human cell lines (Janknecht et al., 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with the recombinant vaccinia virus are loaded onto Ni²⁺ nitriloacetic acid-agarose column and histidine-tagged proteins can be selectively eluted with imidazole-containing buffers. Additional fusion proteins may be generated through the techniques of gene-shuffling, motif-shuffling, exon-shuffling, and/or codon-shuffling (collectively referred to as "DNA shuffling"). DNA shuffling may be employed to modulate the activities of polypeptides of the invention, such methods can be used to generate polypeptides with altered activity, as well as agonists and antagonists of the polypeptides. See, generally, U.S. Patent Nos. 5,605,793; 5,811,238; 5,830,721; 5,834, 252; and 5,837,458, and Patten et al., Curr. Opinion Biotechnol. 8:724-33 (1997); Harayama, Trends Biotechnol. 16(2):76-82 (1998); Hansson, et al., J. Mol. Biol. 287:265-76 (1999); and Lorenzo and Blasco, Biotechniques 24(2):308-13 (1998).

Antibodies of the invention include, but are not limited to, polyclonal, monoclonal, multispecific, human, humanized or chimeric antibodies, single chain antibodies, Fab fragments, F(ab') fragments, fragments produced by a Fab expression library, anti-idiotypic (anti-Id) antibodies (including, e.g., anti-Id antibodies to antibodies of the invention), and epitope-binding fragments of any of the above. The term "antibody," as used herein, refers to immunoglobulin molecules and immunologically active portions of immunoglobulin molecules, i.e., molecules that contain an antigen binding site that immunospecifically binds an antigen. The immunoglobulin molecules of the invention can be of any type (e.g., IgG, IgE, IgM, IgD, IgA and IgY), class (e.g., IgGI, IgG2, IgG3, IgG4, IgAI and IgA2) or subclass of immunoglobulin molecule. In addition, in the context of the present invention, the term "antibody" shall also encompass alternative molecules having the same function, e.g. aptamers and/or CDRs grafted onto alternative peptidic or non-peptidic frames. In some embodiments the antibodies are human antigen-binding antibody fragments and include, but are not limited to, Fab, Fab' and F(ab')2, Fd, single-chain Fvs (scFv), single-chain antibodies, disulfide-linked Fvs (sdFv) and fragments comprising either a VL or VH domain. Antigen-binding antibody fragments, including single-chain antibodies, may comprise the variable region(s) alone or in combination with the entirety or a portion of the following: hinge region, CHI, CH2, and CH3 domains. Also included in the invention are antigen-binding fragments also comprising any combination of variable region(s) with a hinge region, CHI, CH2, and CH3 domains. The antibodies of the invention may be from any animal origin including birds and mammals. In some embodiments, the antibodies are human, murine (e.g., mouse and rat), donkey, ship rabbit, goat, guinea pig, camel, shark, horse, or chicken. As used herein, "human" antibodies include antibodies having the amino acid sequence of a human immunoglobulin and include antibodies isolated from human immunoglobulin libraries or from animals transgenic for one or more human immunoglobulin and that do not express endogenous immunoglobulins, as described infra and, for example in, U.S. Patent No. 5,939,598 by Kucherlapati et al. The antibodies of the present invention may be monospecific, bispecific, trispecific or of greater multi specificity. Multispecific antibodies may be specific for different epitopes of a polypeptide or may be specific for both a polypeptide as well as for a heterologous epitope, such as a heterologous polypeptide or solid support material. See, e.g., PCT publications WO 93/17715; WO 92/08802; WO 91/00360; WO 92/05793; Tutt, et al., J. Immunol. 147:60-69 (1991); U.S. Patent Nos. 4, 474,893; 4,714,681; 4,925,648; 5,573,920; 5,601,819; Kostelny et al., J. Immunol. 148:1547-1553 (1992).

Antibodies of the present invention may be described or specified in terms of the epitope(s) or portion(s) of a polypeptide which they recognize or specifically bind. The epitope(s) or polypeptide portion(s) may be specified as described herein, e.g., by N-terminal and C-terminal positions, by size in contiguous amino acid residues. Antibodies may also be described or specified in terms of their cross-reactivity. Antibodies that do not bind any other analog, ortholog, or homolog of a polypeptide of the present invention are included. Antibodies that bind polypeptides with at least 95%, at least 90%, at least 85%, at least 80%, at least 75%, at least 70%, at least 65%, at least 60%, at least 55%, and at least 50% identity (as calculated using methods known in the art and described herein) to a polypeptide are also included in the present invention. In specific embodiments, antibodies of the present invention cross-react with murine, rat and/or rabbit homologs of human proteins and the corresponding epitopes thereof. Antibodies that do not bind polypeptides with less than 95%, less than 90%, less than 85%, less than 80%, less than 75%, less than 70%, less than 65%, less than 60%. less than 55%, and less than 50% identity (as calculated using methods known in the art and described herein) to a polypeptide are also included in the present invention.

Antibodies may also be described or specified in terms of their binding affinity to a polypeptide. Antibodies may act as agonists or antagonists of the recognized polypeptides. The invention also features receptor-specific antibodies which do not prevent ligand binding but prevent receptor activation. Receptor activation (i.e., signaling) may be determined by techniques described herein or otherwise known in the art. For example, receptor activation can be determined by detecting the phosphorylation (e.g., tyrosine or serine/threonine) of the receptor or of one of its down-stream substrates by immunoprecipitation followed by western blot analysis (for example, as described supra). In specific embodiments, antibodies are provided that inhibit ligand activity or receptor activity by at least 95%, at least 90%, at least 85%, at least 80%, at least 75%, at least 70%, at least 60%, or at least 50% of the activity in absence of the antibody.

The invention also features receptor-specific antibodies which both prevent ligand binding and receptor activation as well as antibodies that recognize the receptor-ligand complex. Likewise, encompassed by the invention are antibodies which bind the ligand, thereby preventing receptor activation, but do not prevent the ligand from binding the receptor. The antibodies may be specified as agonists, antagonists or inverse agonists for biological activities comprising the specific biological activities of the peptides disclosed herein. The above antibody agonists can be made using methods known in the art. See, e.g., PCT publication WO 96/40281; U.S. Patent No. 5,811, 097; Deng et al., Blood 92(6):1981-1988 (1998); Chen et al., Cancer Res. 58(16):3668-3678 (1998); Harrop et al., J. Immunol. 161(4):1786-1794 (1998); Zhu et al., Cancer Res. 58(15):3209-3214 (1998); Yoon et al., J. Immunol. 160(7):3170-3179 (1998); Prat et al., J. Cell. Sci. III(Pt2) :237-247 (1998); Pitard et al., J. Immunol. Methods 205(2):177-190 (1997); Liautard et al., Cytokine 9(4):233-241 (1997); Carlson et al., J. Biol. Chem. 272(17):11295-11301 (1997); Taryman et al., Neuron 14(4):755-762 (1995); Muller et al., Structure 6(9):1153-1167 (1998); Bartunek et al., Cytokine 8(I):14-20 (1996).

As discussed in more detail below, the antibodies may be used either alone or in combination with other compositions. The antibodies may further be recombinantly fused to a heterologous polypeptide at the N-or C-terminus or chemically conjugated (including covalently and non-covalently conjugations) to polypeptides or other compositions. For example, antibodies of the present invention may be recombinantly fused or conjugated to molecules useful as labels in detection assays and effector molecules such as heterologous polypeptides, drugs, radionuclides, or toxins. See, e.g., PCT publications WO 92/08495; WO 91/14438; WO 89/12624; U.S. Patent No. 5,314,995; and EP 396, 387. The antibodies as defined for the present invention include derivatives that are modified, i. e, by the covalent attachment of any type of molecule to the antibody such that covalent attachment does not prevent the antibody from generating an anti-idiotypic response. For example, but not by way of limitation, the antibody derivatives include antibodies that have been modified, e.g., by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

The antibodies of the present invention may be generated by any suitable method known in the art. Polyclonal antibodies to an antigen-of-interest can be produced by various procedures well known in the art. For example, a polypeptide of the invention can be administered to various host animals including, but not limited to, rabbits, mice, rats, etc. to induce the production of sera containing polyclonal antibodies specific for the antigen.

Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvurn. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al. , Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art.

Antibody fragments which recognize specific epitopes may be generated by known techniques. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CHl domain of the heavy chain.

For example, the antibodies can also be generated using various phage display methods known in the art. In phage display methods, functional antibody domains are displayed on the surface of phage particles which carry the polynucleotide sequences encoding them. In a particular embodiment, such phage can be utilized to display antigen binding domains expressed from a repertoire or combinatorial antibody library (e.g., human or murine). Phage expressing an antigen binding domain that binds the antigen of interest can be selected or identified with antigen, e.g., using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Examples of phage display methods that can be used to make the antibodies of the present invention include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT application No. PCT/GB91/01134; PCT publications WO 90/02809; WO 91/10737; WO 92/01047; WO 92/18619; WO 93/11236; WO 95/15982; WO 95/20401; and U.S. Patent Nos. 5, 698,426; 5,223,409; 5,403,484; 5,580,717; 5,427,908; 5,750,753; 5,821, 047; 5,571, 698; 5,427,908; 5,516,637; 5,780,225; 5,658,727; 5,733,743 and 5,969,108. As described in these references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')2 fragments can also be employed using methods known in the art such as those disclosed in PCT publication WO 92/22324; Mullinax. et al., BioTechniques 12(6):864-869 (1992); and Sawai et al., AJRI 34:26-34 (1995); and Better et al., Science 240:1041-1043 (1988).

Examples of techniques which can be used to produce single-chain Fvs and antibodies include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988). For some uses, including in vivo use of antibodies in humans and in vitro detection assays, it may be preferable to use chimeric, humanized, or human antibodies. A chimeric antibody is a molecule in which different portions of the antibody are derived from different animal species, such as antibodies having a variable region derived from a murine monoclonal antibody and a human immunoglobulin constant region. Methods for producing chimeric antibodies are known in the art. See e.g., Morrison, Science 229:1202 (1985); Oi et al., BioTechniques 4:214 (1986); Gillies et al., (1989) J. Immunol. Methods 125:191-202; U.S. Patent Nos. 5,807,715; 4,816,567; and 4,816397. Humanized antibodies are antibody molecules from non-human species antibody that binds the desired antigen having one or more complementarity determining regions (CDRs) from the non-human species and a framework regions from a human immunoglobulin molecule. Often, framework residues in the human framework regions will be substituted with the corresponding residue from the CDR donor antibody to alter, and/or improve, antigen binding. These framework substitutions are identified by methods well known in the art, e.g., by modelling of the interactions of the CDR and framework residues to identify framework residues important for antigen binding and sequence comparison to identify unusual framework residues at particular positions. (See, e.g., Queen et al., U.S. Patent No. 5,585,089; Riechmann et al., Nature 332:323 (1988).) Antibodies can be humanized using a variety of techniques known in the art including, for example, CDR-grafting (EP 239,400; PCT publication WO 91/09967; U.S. Patent Nos. 5,225,539; 5,530,101; and 5,585,089), veneering or resurfacing (EP 592, 106; EP 519,596; Padlan, Molecular Immunology 28(4/5):489-498 (1991); Studnicka et al., Protein Engineering 7(6):805-814 (1994); Roguska. et al., PNAS 91:969-973 (1994)), and chain shuffling (U.S. Patent No. 5,565,332). Completely human antibodies are particularly desirable for therapeutic treatment of human patients. Human antibodies can be made by a variety of methods known in the art including phage display methods described above using antibody libraries derived from human immunoglobulin sequences. See also, U.S. Patent Nos. 4,444,887 and 4,716, 111; and PCT publications WO 98/46645, WO 98/50433, WO 98/24893, WO 98/16654, WO 96/34096, WO 96/33735, and WO 91/10741.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, e.g., all or a portion of a polypeptide of the invention. Monoclonal antibodies directed against the antigen can be obtained from the immunized, transgenic mice using conventional hybridoma technology. The human immunoglobulin transgenes harboured by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar, Int. Rev. Immurnol. 13:65-93 (1995). For a detailed discussion of this technology for producing human antibodies and human monoclonal antibodies and protocols for producing such antibodies, see, e. g., PCT publications WO 98/24893; WO 92/01047; WO 96/34096; WO 96/33735; European Patent No. 0 598 877; U.S. Patent Nos. 5,413,923; 5,625,126; 5,633,425; 5,569, 825; 5, 661,016; 5,545,806; 5,814,318; 5,885,793; 5,916,771; and 5,939,598. In addition, companies such as Abgenix, Inc. (Freemont, CA) and Genpharm (San Jose, CA) can be engaged to provide human antibodies directed against a selected antigen using technology similar to that described above. Completely human antibodies which recognize a selected epitope can be generated using a technique referred to as "guided selection." In this approach a selected non-human monoclonal antibody, e.g., a mouse antibody, is used to guide the selection of a completely human antibody recognizing the same epitope. (Jespers et al., Bio/technology 12:899-903 (1988)). Furthermore, antibodies can be utilized to generate anti-idiotype antibodies that "mimic" polypeptides using techniques well known to those skilled in the art. (See, e.g., Greenspan & Bona, FASEB J. 7(5):437-444; (1989) and Nissinoff, J. Immunol. 147(8):2429-2438 (1991)). For example, antibodies which bind to and competitively inhibit polypeptide multimerization. and/or binding of a polypeptide to a ligand can be used to generate anti-idiotypes that "mimic" the polypeptide multimerization. and/or binding domain and, as a consequence, bind to and neutralize polypeptide and/or its ligand. Such neutralizing anti-idiotypes or Fab fragments of such anti-idiotypes can be used in therapeutic regimens to neutralize polypeptide ligand. For example, such anti-idiotypic antibodies can be used to bind a polypeptide and/or to bind its ligands/receptors, and thereby block its biological activity. Polynucleotides encoding antibodies, comprising a nucleotide sequence encoding an antibody are also encompassed. These polynucleotides may be obtained, and the nucleotide sequence of the polynucleotides determined, by any method known in the art. For example, if the nucleotide sequence of the antibody is known, a polynucleotide encoding the antibody may be assembled from chemically synthesized oligonucleotides (e.g., as described in Kutmeier et al., BioTechniques 17:242 (1994)), which, briefly, involves the synthesis of overlapping oligonucleotides containing portions of the sequence encoding the antibody, annealing and ligating of those oligonucleotides, and then amplification of the ligated oligonucleotides by PCR.

The amino acid sequence of the heavy and/or light chain variable domains may be inspected to identify the sequences of the complementarity determining regions (CDRs) by methods that are well known in the art, e.g., by comparison to known amino acid sequences of other heavy and light chain variable regions to determine the regions of sequence hypervariability. Using routine recombinant DNA techniques, one or more of the CDRs may be inserted within framework regions, e.g., into human framework regions to humanize a non-human antibody, as described supra. The framework regions may be naturally occurring or consensus framework regions, and in some embodiments, human framework regions (see, e.g., Chothia et al., J. Mol. Biol. 278: 457-479 (1998) for a listing of human framework regions). In some embodiments, the polynucleotide generated by the combination of the framework regions and CDRs encodes an antibody that specifically binds a polypeptide. In some embodiments, as discussed supra, one or more amino acid substitutions may be made within the framework regions, and, in some embodiments, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present description and within the skill of the art.

In addition, techniques developed for the production of "chimeric antibodies" (Morrison et al., Proc. Natl. Acad. Sci. 81:851-855 (1984); Neuberger et al., Nature 312:604-608 (1984); Takeda et al., Nature 314:452-454 (1985)) by splicing genes from a mouse antibody molecule of appropriate antigen specificity together with genes from a human antibody molecule of appropriate biological activity can be used. As described supra, a chimeric antibody is a molecule in which different portions are derived from different animal species, such as those having a variable region derived from a murine mAb and a human immunoglobulin constant region, e.g., humanized antibodies.

Alternatively, techniques described for the production of single chain antibodies (U.S. Patent No. 4,946,778; Bird, Science 242:423-42 (1988); Huston et al., Proc. Natl. Acad. Sci. USA 85:5879-5883 (1988); and Ward et al., Nature 334:544-54 (1989)) can be adapted to produce single chain antibodies. Single chain antibodies are formed by linking the heavy and light chain fragments of the Fv region via an amino acid bridge, resulting in a single chain polypeptide. Techniques for the assembly of functional Fv fragments in E. coli may also be used (Skerra et al., Science 242:1038-1041 (1988)). The present invention encompasses antibodies recombinantly fused or chemically conjugated (including both covalently and non-covalently conjugations) to a polypeptide (or portion thereof, in some embodiments, at least 10, 20, 30, 40, 50, 60, 70, 80, 90 or 100 amino acids of the polypeptide) to generate fusion proteins. The fusion does not necessarily need to be direct, but may occur through linker sequences. The antibodies may be specific for antigens other than polypeptides (or portion thereof, in some embodiments, at least 10, 20, 30, 40, 50, 60, 70, 80, 90 or 100 amino acids of the polypeptide). Further, an antibody or fragment thereof may be conjugated to a therapeutic moiety, for instance to increase their therapeutical activity. The conjugates can be used for modifying a given biological response, the therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, a-interferon, B-interferon, nerve growth factor, platelet derived growth factor, tissue plasminogen activator, an apoptotic agent, e.g., TNF-alpha, TNF-beta, AIM I (See, International Publication No. WO 97/33899), AIM 11 (See, International Publication No. WO 97/34911), Fas Ligand (Takahashi et aL, Int. Immunol., 6:1567-1574 (1994)), VEGI (See, International Publication No. WO 99/23105), a thrombotic agent or an anti-angiogenic agent, e.g., angiostatin or endostatin; or, biological response modifiers such as, for example, lymphokines, interleukin-1 ("IL-I"), interleukin-2 ("IL-2"), interleukin-6 ("IL-6"), granulocyte macrophage colony stimulating factor ("GM-CSF"), granulocyte colony stimulating factor ("G-CSF"), or other growth factors. Techniques for conjugating such therapeutic moiety to antibodies are well known, see, e.g., Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev. 62:119-58 (1982). Alternatively, an antibody can be conjugated to a second antibody to form an antibody heteroconjugate as described by Segal in U.S. Patent No. 4,676, 980.

The present invention is also directed to antibody-based therapies which involve administering antibodies of the invention to an animal, in some embodiments, a mammal, for example a human, patient to treat neurodegenerative diseases. Therapeutic compounds include, but are not limited to, antibodies (including fragments, analogs and derivatives thereof as described herein) and nucleic acids encoding antibodies of the invention (including fragments, analogs and derivatives thereof and anti-idiotypic antibodies as described herein). Antibodies of the invention may be provided in pharmaceutically acceptable compositions as known in the art or as described herein.

The invention also provides methods for treating neurodegenerative diseases in a subject by increasing the enzymatic activity of HDAC6 by administration to the subject of an effective amount of an compound or pharmaceutical composition comprising an agnostic compound of HDAC6. In some embodiments, said inhibitory compound is a small molecule, an antibody or a siRNA. In an embodiment, the compound is substantially purified (e.g., substantially free from substances that limit its effect or produce undesired side-effects). The subject is in some embodiments, an animal, including but not limited to animals such as cows, pigs, horses, chickens, cats, dogs, etc., and is in some embodiments, a mammal, for example human.

Formulations and methods of administration that can be employed when the compound comprises a nucleic acid or an immunoglobulin are described above; additional appropriate formulations and routes of administration can be selected from among those described herein below.

Various delivery systems are known and can be used to administer a compound, e.g., encapsulation in liposomes, microparticles, microcapsules, recombinant cells capable of expressing the compound, receptor-mediated endocytosis (see, e. g., Wu and Wu, J. Biol. Chem. 262:4429-4432 (1987)), construction of a nucleic acid as part of a retroviral or other vector, etc. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compounds or compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compounds or compositions of the invention locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers.

In another embodiment, the compound or composition can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid.) In yet another embodiment, the compound or composition can be delivered in a controlled release system. In one embodiment, a pump may be used (see Langer, supra; Sefton, CRC Crit. Ref, Biomed. Eng. 14:201 (1987); Buchwald et al., Surgery 88:507 (1980); Saudek et al., N. Engl. J. Med. 321:574 (1989)). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., Macromol. Sci. Rev. Macromol. Chem. 23:61 (1983); see also Levy et al., Science 228:190 (1985); During et al., Ann. Neurol. 25:351 (1989); Howard et al., J. Neurosurg. 71:105 (1989)). In yet another embodiment, a controlled release system can be placed in proximity of the therapeutic target, i.e., the brain, thus requiring only a fraction of the systemic dose (see, e.g. , Goodson, in Medical Applications of Controlled Release, supra, vol. 2, pp. 115-13 8 (1984)). Other controlled release systems are discussed in the review by Langer (Science 249:1527-1533 (1990)).

The present invention also provides pharmaceutical compositions for use in the treatment of neurodegenerative diseases by increasing the enzymatic activity of HDAC6. Such compositions comprise a therapeutically effective amount of an inhibitory compound, and a pharmaceutically acceptable carrier. In a specific embodiment, the term "pharmaceutically acceptable" means approved by a regulatory agency of the Federal or a state government or listed in the U. S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, tale, sodium chloride, driied skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, in some embodiments, in purified form, together with a suitable amount of carrier so as to provide the form for proper administration to the patient. The formulation should suit the mode of administration.

In an embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anaesthetic such as lidocaine to ease pain at the site of the injection.

Generally, the ingredients are supplied either separately or mixed together in unit dosage form, for example, as a dry lyophilized powder or water free concentrate in a hermetically scaled container such as an ampoule or sachette indicating the quantity of active agent.

Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the invention can be formulated as neutral or salt forms.

Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc. The amount of the compound which will be effective in the treatment, inhibition and prevention of a disease or disorder associated with aberrant expression and/or activity of a polypeptide of the invention can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each patient's circumstances.

Effective doses may be extrapolated from dose-response curves derived from *in vitro* or animal model test systems. For antibodies, the dosage administered to a patient is typically 0.1 mg/kg to 100 mg/kg of the patient's body weight. In some embodiments, the dosage administered to a patient is between 0.1 mg/kg and 20 mg/kg of the patient's body weight, for example1 mg/kg to 10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the invention may be reduced by enhancing uptake and tissue penetration (e.g., into the brain) of the antibodies by modifications such as, for example, lipidation.

Also encompassed is a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The antibodies as encompassed herein may also be chemically modified derivatives which may provide additional advantages such as increased solubility, stability and circulating time of the polypeptide, or decreased immunogenicity (see U. S. Patent No. 4,179,337). The chemical moieties for derivatisation may be selected from water soluble polymers such as polyethylene glycol, ethylene glycol/propylene glycol copolymers, carboxymethylcellulose, dextran, polyvinyl alcohol and the like. The antibodies may be modified at random positions within the molecule, or at predetermined positions within the molecule and may include one, two, three or more attached chemical moieties. The polymer may be of any molecular weight, and may be branched or unbranched. For polyethylene glycol, the preferred molecular weight is between about 1 kDa and about 100000 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog). For example, the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,600, 17,500, 18,000, 18,500, 19,000, 19,500, 20,000, 25,000, 30,000, 35,000, 40,000, 50,000, 55,000, 60,000, 65,000, 70,000, 75,000, 80,000, 85,000, 90,000, 95,000, or 100,000 kDa. As noted above, the polyethylene glycol may have a branched structure. Branched polyethylene glycols are described, for example, in U. S. Patent No. 5,643, 575; Morpurgo et al., Appl. Biochem. Biotechnol. 56:59-72 (1996); Vorobjev et al., Nucleosides Nucleotides 18:2745-2750 (1999); and Caliceti et al., Bioconjug. Chem. 10:638-646 (1999). The polyethylene glycol molecules (or other chemical moieties) should be attached to the protein with consideration of effects on functional or antigenic domains of the protein. There are a number of attachment methods available to those skilled in the art, e.g., EP 0 401 384 (coupling PEG to G-CSF), see also Malik et al., Exp. Hematol. 20:1028-1035 (1992) (reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues glutamic acid residues and the C-terminal amino acid residue. Sulfhydryl groups may also be used as a reactive group for attaching the polyethylene glycol molecules. Preferred for therapeutic purposes is attachment at an amino group, such as attachment at the N-terminus or lysine group. As suggested above, polyethylene glycol may be attached to proteins via linkage to any of a number of amino acid residues. For example, polyethylene glycol can be linked to proteins via covalent bonds to lysine, histidine, aspartic acid, glutamic acid, or cysteine residues. One or more reaction chemistries may be employed to attach polyethylene glycol to specific amino acid residues (e.g., lysine, histidine, aspartic acid, glutamic acid, or cysteine) of the protein or to more than one type of amino acid residue (e.g., lysine, histidine, aspartic acid, glutamic acid, cysteine and combinations thereof) of the protein. As indicated above, pegylation of the proteins of the invention may be accomplished by any number of means. For example, polyethylene glycol may be attached to the protein either directly or by an intervening linker. Linkerless systems for attaching polyethylene glycol to proteins are described in Delgado et al., Crit. Rev. Thera. Drug Carrier Sys. 9:249-304 (1992); Francis et al., Intern. J. of Hematol. 68:1-18 (1998); U.S. Patent No. 4,002,53 1; U.S. Patent No. 5,349,052; WO 95/06058; and WO 98/32466.

"siRNA" or "small-interfering ribonucleic acid" according to the invention has the meanings known in the art, including the following aspects. The siRNA consists of two strands of ribonucleotides which hybridize along a complementary region under physiological conditions. The strands are normally separate. Because of the two strands have separate roles in a cell, one strand is called the "anti-sense" strand, also known as the "guide" sequence, and is used in the functioning RISC complex to guide it to the correct mRNA for cleavage. This use of "anti-sense", because it relates to an RNA compound, is different from the antisense target DNA compounds referred to elsewhere in this specification. The other strand is known as the "anti-guide" sequence and because it contains the same sequence of nucleotides as the target sequence, it is also known as the sense strand. The strands may be joined by a molecular linker in certain embodiments. The individual ribonucleotides may be unmodified naturally occurring ribonucleotides, unmodified naturally occurring deoxyribonucleotides or they may be chemically modified or synthetic as described elsewhere herein. In some embodiments, the siRNA molecule is substantially identical with at least a region of the coding sequence of the target gene to enable down-regulation of the gene. In some embodiments, the degree of identity between the sequence of the siRNA molecule and the targeted region of the gene is at least 60% sequence identity, in some embodiments at least 75% sequence identity, for instance at least 85% identity, 90% identity, at least 95% identity, at least 97%, or at least 99% identity. Calculation of percentage identities between different amino acid/polypeptide/nucleic acid sequences may be carried out as follows. A multiple alignment is first generated by the ClustalX program (pairwise parameters: gap opening 10.0, gap extension 0.1, protein matrix Gonnet 250, DNA matrix IUB; multiple parameters: gap opening 10.0, gap extension 0.2, delay divergent sequences 30%, DNA transition weight 0.5, negative matrix off, protein matrix gonnet series, DNA weight IUB; Protein gap parameters, residue-specific penalties on, hydrophilic penalties on, hydrophilic residues GPSNDQERK, gap separation distance 4, end gap separation off). The percentage identity is then calculated from the multiple alignment as (N/T)* 100, where N is the number of positions at which the two sequences share an identical residue, and T is the total number of positions compared. Alternatively, percentage identity can be calculated as (N/S)* 100 where S is the length of the shorter sequence being compared. The amino acid/polypeptide/nucleic acid sequences may be synthesised de novo, or may be native amino acid/polypeptide/nucleic acid sequence, or a derivative thereof. A substantially similar nucleotide sequence will be encoded by a sequence which hybridizes to any of the nucleic acid sequences referred to herein or their complements under stringent conditions. By stringent conditions, we mean the nucleotide hybridises to filter-bound DNA or RNA in 6x sodium chloride/sodium citrate (SSC) at approximately 45°C followed by at least one wash in 0.2x SSC/0.l% SDS at approximately 5-65°C. Alternatively, a substantially similar polypeptide may differ by at least 1, but less than 5, 10, 20, 50 or 100 amino acids from the peptide sequences according to the present invention Due to the degeneracy of the genetic code, it is clear that any nucleic acid sequence could be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequences which are altered by the substitution of different codons that encode an amino acid with a side chain of similar biophysical properties to the amino acid it substitutes, to produce a conservative change. For example small non-polar, hydrophobic amino acids include glycine, alanine, leucine, isoleucine, valine, proline, and methionine; large non-polar, hydrophobic amino acids include phenylalanine, tryptophan and tyrosine; the polar neutral amino acids include serine, threonine, cysteine, asparagine and glutamine; the positively charged (basic) amino acids include lysine, arginine and histidine; and the negatively charged (acidic) amino acids include aspartic acid and glutamic acid. The accurate alignment of protein or DNA sequences is a complex process, which has been investigated in detail by a number of researchers. Of particular importance is the trade-off between optimal matching of sequences and the introduction of gaps to obtain such a match. In the case of proteins, the means by which matches are scored is also of significance. The family of PAM matrices (e.g., Dayhoff, M. et al., 1978, Atlas of protein sequence and structure, Natl. Biomed. Res. Found.) and BLOSUM matrices quantify the nature and likelihood of conservative substitutions and are used in multiple alignment algorithms, although other, equally applicable matrices will be known to those skilled in the art. The popular multiple alignment program ClustalW, and its windows version ClustalX (Thompson et al., 1994, Nucleic Acids Research, 22, 4673-4680; Thompson et al., 1997, Nucleic Acids Research, 24, 4876-4882) are efficient ways to generate multiple alignments of proteins and DNA. Frequently, automatically generated alignments require manual alignment, exploiting the trained user's knowledge of the protein family being studied, e.g., biological knowledge of key conserved sites. One such alignment editor programs is Align (http://www.gwdg. de/dhepper/download/; Hepperle, D., 2001: Multicolor Sequence Alignment Editor. Institute of Freshwater Ecology and Inland Fisheries, 16775 Stechlin, Germany), although others, such as JalView or Cinema are also suitable. Calculation of percentage identities between proteins occurs during the generation of multiple alignments by Clustal. However, these values need to be recalculated if the alignment has been manually improved, or for the deliberate comparison of two sequences. Programs that calculate this value for pairs of protein sequences within an alignment include PROTDIST within the PHYLIP phylogeny package (Felsenstein; http://evolution.gs. washington.edu/ phylip.html) using the "Similarity Table" option as the model for amino acid substitution (P). For DNA/RNA, an identical option exists within the DNADIST program of PHYL1P. The dsRNA molecules in accordance with the present invention comprise a double-stranded region which is substantially identical to a region of the mRNA of the target gene. A region with 100% identity to the corresponding sequence of the target gene is suitable. This state is referred to as "fully complementary". However, the region may also contain one, two or three mismatches as compared to the corresponding region of the target gene, depending on the length of the region of the mRNA that is targeted, and as such may be not fully complementary. In an embodiment, the RNA molecules of the present invention specifically target one given gene. In order to only target the desired mRNA, the siRNA reagent may have 100% homology to the target mRNA and at least 2 mismatched nucleotides to all other genes present in the cell or organism. Methods to analyze and identify siRNAs with sufficient sequence identity in order to effectively inhibit expression of a specific target sequence are known in the art. Sequence identity may be optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group).

The length of the region of the siRNA complementary to the target, in accordance with the present invention, may be from 10 to 100 nucleotides, 12 to 25 nucleotides, 14 to 22 nucleotides or 15, 16, 17 or 18 nucleotides. Where there are mismatches to the corresponding target region, the length of the complementary region is generally required to be somewhat longer. In an embodiment, the inhibitor is a siRNA molecule and comprises between approximately 5bp and 50 bp, in some embodiments, between 10 bp and 35 bp, or between 15 bp and 30 bp, for instance between 18 bp and 25bp. In some embodiments, the siRNA molecule comprises more than 20 and less than 23 bp.

Because the siRNA may carry overhanging ends (which may or may not be complementary to the target), or additional nucleotides complementary to itself but not the target gene, the total length of each separate strand of siRNA may be 10 to 100 nucleotides, 15 to 49 nucleotides, 17 to 30 nucleotides or 19 to 25 nucleotides.

The phrase "each strand is 49 nucleotides or less" means the total number of consecutive nucleotides in the strand, including all modified or unmodified nucleotides, but not including any chemical moieties which may be added to the 3' or 5' end of the strand. Short chemical moieties inserted into the strand are not counted, but a chemical linker designed to join two separate strands is not considered to create consecutive nucleotides.

The phrase "a 1 to 6 nucleotide overhang on at least one of the 5' end or 3' end" refers to the architecture of the complementary siRNA that forms from two separate strands under physiological conditions. If the terminal nucleotides are part of the double-stranded region of the siRNA, the siRNA is considered blunt ended. If one or more nucleotides are unpaired on an end, an overhang is created. The overhang length is measured by the number of overhanging nucleotides. The overhanging nucleotides can be either on the 5' end or 3' end of either strand.

The siRNA according to the present invention display a high in vivo stability and may be particularly suitable for oral delivery by including at least one modified nucleotide in at least one of the strands. Thus the siRNA according to the present invention contains at least one modified or non-natural ribonucleotide. A lengthy description of many known chemical modifications are set out in published PCT patent application WO 200370918. Suitable modifications for delivery include chemical modifications can be selected from among: a) a 3' cap; b) a 5' cap,c) a modified internucleoside linkage; or d) a modified sugar or base moiety.

Suitable modifications include, but are not limited to modifications to the sugar moiety (i.e. the 2' position of the sugar moiety, such as for instance 2'-O-(2-methoxyethyl) or 2'-MOE) (Martin et al., Helv. Chim. Acta, 1995, 78, 486-504) i.e., an alkoxyalkoxy group) or the base moiety (i.e. a non-natural or modified base which maintains ability to pair with another specific base in an alternate nucleotide chain). Other modifications include so-called 'backbone' modifications including, but not limited to, replacing the phosphoester group (connecting adjacent ribonucleotides) with for instance phosphorothioates, chiral phosphorothioates or phosphorodithioates.

End modifications sometimes referred to herein as 3' caps or 5' caps may be of significance. Caps may consist of simply adding additional nucleotides, such as "T-T" which has been found to confer stability on a siRNA. Caps may consist of more complex chemistries which are known to those skilled in the art.

Design of a suitable siRNA molecule is a complicated process, and involves very carefully analysing the sequence of the target mRNA molecule. On exemplary method for the design of siRNA is illustrated in WO2005/059132. Then, using considerable inventive endeavour, the inventors have to choose a defined sequence of siRNA which has a certain composition of nucleotide bases, which would have the required affinity and also stability to cause the RNA interference.

The siRNA molecule may be either synthesised de novo, or produced by a micro-organism. For example, the siRNA molecule may be produced by bacteria, for example, E. coli. Methods for the synthesis of siRNA, including siRNA containing at least one modified or non-natural ribonucleotides are well known and readily available to those of skill in the art. For example, a variety of synthetic chemistries are set out in published PCT patent applications WO2005021749 and WO200370918. The reaction may be carried out in solution or, in some embodiments, on solid phase or by using polymer supported reagents, followed by combining the synthesized RNA strands under conditions, wherein a siRNA molecule is formed, which is capable of mediating RNAi.

It should be appreciated that siNAs (small interfering nucleic acids) may comprise uracil (siRNA) or thyrimidine (siDNA). Accordingly the nucleotides U and T, as referred to above, may be interchanged. However it is preferred that siRNA is used.

Gene-silencing molecules, i.e. inhibitors, used according to the invention are in some embodiments, nucleic acids (e.g. siRNA or antisense or ribozymes). Such molecules may (but not necessarily) be ones, which become incorporated in the DNA of cells of the subject being treated. Undifferentiated cells may be stably transformed with the gene-silencing molecule leading to the production of genetically modified daughter cells (in which case regulation of expression in the subject may be required, e.g. with specific transcription factors, or gene activators).

The gene-silencing molecule may be either synthesised *de novo*, and introduced in sufficient amounts to induce gene-silencing (e.g. by RNA interference) in the target cell. Alternatively, the molecule may be produced by a micro-organism, for example, E. coli, and then introduced in sufficient amounts to induce gene silencing in the target cell. Alternatively, the molecule may be produced using, for example, lentiviral vectors that are typically packaged using a three-plasmid system in a human cell line such as the HEK293: the first vector encodes the viral genome and is transcribed into RNA and packaged into the virus particle, the second vector encodes the viral glycoprotein, and the third vector encodes the viral structural proteins (GAG) and viral enzymes (POL: consisting of reverse transcriptase, protease, and integrase).

The molecule may be produced by a vector harbouring a nucleic acid that encodes the gene-silencing sequence. The vector may comprise elements capable of controlling and/or enhancing expression of the nucleic acid. The vector may be a recombinant vector. The vector may for example comprise plasmid, cosmid, phage, or virus DNA. In addition to, or instead of using the vector to synthesise the gene-silencing molecule, the vector may be used as a delivery system for transforming a target cell with the gene silencing sequence.

The recombinant vector may also include other functional elements. For instance, recombinant vectors can be designed such that the vector will autonomously replicate in the target cell. In this case, elements that induce nucleic acid replication may be required in the recombinant vector. Alternatively, the recombinant vector may be designed such that the vector and recombinant nucleic acid molecule integrates into the genome of a target cell. In this case nucleic acid sequences, which favour targeted integration (e.g. by homologous recombination) are desirable. Recombinant vectors may also have DNA coding for genes that may be used as selectable markers in the cloning process.

The recombinant vector may also comprise a promoter or regulator or enhancer to control expression of the nucleic acid as required. Tissue specific promoter/enhancer elements may be used to regulate expression of the nucleic acid in specific cell types, for example, endothelial cells. The promoter may be constitutive or inducible.

Alternatively, the gene silencing molecule may be administered to a target cell or tissue in a subject with or without it being incorporated in a vector. For instance, the molecule may be incorporated within a liposome or virus particle (e.g. a retrovirus, herpes virus, pox virus, vaccina virus, adenovirus, lentivirus and the like).

Alternatively a "naked" siRNA or antisense molecule may be inserted into a subject's cells by a suitable means e.g. direct endocytotic uptake.

The gene silencing molecule may also be transferred to the cells of a subject to be treated by either transfection, infection, microinjection, cell fusion, protoplast fusion or ballistic bombardment. For example, transfer may be by: ballistic transfection with coated gold particles; liposomes containing a siNA molecule; viral vectors comprising a gene silencing sequence or means of providing direct nucleic acid uptake (e.g. endocytosis) by application of the gene silencing molecule directly.

In an embodiment of the present invention siNA molecules may be delivered to a target cell (whether in a vector or "naked") and may then rely upon the host cell to be replicated and thereby reach therapeutically effective levels. When this is the case the siNA is in some embodiments, incorporated in an expression cassette that will enable the siNA to be transcribed in the cell and then interfere with translation (by inducing destruction of the endogenous mRNA coding the targeted gene product). Inhibitors according to any embodiment of the present invention may be used in a monotherapy (e.g. use of siRNAs alone). However it will be appreciated that the inhibitors may be used as an adjunct, or in combination with other therapies.

The modulator of PRKD1 may be contained within compositions having a number of different forms depending, in particular on the manner in which the composition is to be used. Thus, for example, the composition may be in the form of a capsule, liquid, ointment, cream, gel, hydrogel, aerosol, spray, micelle, transdermal patch, liposome or any other suitable form that may be administered to a person or animal. It will be appreciated that the vehicle of the composition of the invention should be one which is well tolerated by the subject to whom it is given, and in some embodiments, enables delivery of the inhibitor to the target site.

The agents may be used in a number of ways. For instance, systemic administration may be required in which case the compound may be contained within a composition that may, for example, be administered by injection into the blood stream. Injections may be intravenous (bolus or infusion), subcutaneous, intramuscular or a direct injection into the target tissue (e.g. an intraventricular injection-when used in the brain). The inhibitors may also be administered by inhalation (e.g. intranasally) or even orally (if appropriate).

The agents of the invention may also be incorporated within a slow or delayed release device. Such devices may, for example, be inserted in the body of the subject, and the molecule may be released over weeks or months. Such devices may be particularly advantageous when long term treatment with an agonist of HDAC6 is required and which would normally require frequent administration (e.g. at least daily injection).

It will be appreciated that the amount of an agent that is required is determined by its biological activity and bioavailability which in turn depends on the mode of administration, the physicochemical properties of the molecule employed and whether it is being used as a monotherapy or in a combined therapy. The frequency of administration will also be influenced by the above-mentioned factors and particularly the half-life of the inhibitor within the subject being treated.

Optimal dosages to be administered may be determined by those skilled in the art, and will vary with the particular inhibitor in use, the strength of the preparation, and the mode of administration. Additional factors depending on the particular subject being treated will result in a need to adjust dosages, including subject age, weight, gender, diet, and time of administration.

When the agent is a nucleic acid conventional molecular biology techniques (vector transfer, liposome transfer, ballistic bombardment etc) may be used to deliver the inhibitor to the target tissue.

Known procedures, such as those conventionally employed by the pharmaceutical industry (e.g. in vivo experimentation, clinical trials, etc.), may be used to establish specific formulations for use according to the invention and precise therapeutic regimes (such as daily doses of the gene silencing molecule and the frequency of administration).

Generally, a daily dose of between 0.01 µg/kg of body weight and 0.5 g/kg of body weight of an agonist of HDAC6 may be used for the treatment of neurodegenerative diseases in the subject, depending upon which specific inhibitor is used. When the inhibitor is an siRNA molecule, the daily dose may be between 1 pg/kg of body weight and 100 mg/kg of body weight, in some embodiments, between approximately 10 pg/kg and 10 mg/kg, or between about 50 pg/kg and 1mg/kg.

When the inhibitor (e.g. siNA) is delivered to a cell, daily doses may be given as a single administration (e.g. a single daily injection).

Various assays are known in the art to test dsRNA for its ability to mediate RNAi (see for instance Elbashir et al., Methods 26 (2002), 199-213). The effect of the dsRNA according to the present invention on gene expression will typically result in expression of the target gene being inhibited by at least 10%, 33%, 50%, 90%, 95% or 99% when compared to a cell not treated with the RNA molecules according to the present invention.

Similarly, various assays are well-known in the art to test antibodies for their ability to inhibit the biological activity of their specific targets. The effect of the use of an antibody according to the present invention will typically result in biological activity of their specific target being inhibited by at least 10%, 33%, 50%, 90%, 95% or 99% when compared to a control not treated with the antibody.

The terms "frataxin," "FA," "X25," "CyaY" "FARR," "MGC57199," "FXN" interchangeably refer to nucleic acids and polypeptide polymorphic variants, alleles, mutants, and interspecies homologs that: (1) have an amino acid sequence that has greater than about 90% amino acid sequence identity, for example, 91%, 92%, 93%, 94%, 95%, 96%, 97%, 98% or 99% or greater amino acid sequence identity, preferably over a region of at least about 25, 50, 100, 200, 300, 400, or more amino acids, or over the full-length, to an amino acid sequence encoded by a frataxin nucleic acid (see, e.g., GenBank Accession Nos. NM_000144.4 (isoform 1); NM_181425.2 (isoform 2); NM_001161706.1 (isoform 3)) or to an amino acid sequence of a frataxin polypeptide (see, e.g. GenBank Accession Nos. NP_000135.2 (isoform 1); NP_852090.1 (isoform 2); NP_001155178.1 (isoform 3)); (2) bind to antibodies, e.g., polyclonal antibodies, raised against an immunogen comprising an amino acid sequence of a frataxin polypeptide (e.g., frataxin polypeptides described herein); or an amino acid sequence encoded by a frataxin nucleic acid (e.g., frataxin polynucleotides described herein), and conservatively modified variants thereof; (3) specifically hybridize under stringent hybridization conditions to an anti-sense strand corresponding to a nucleic acid sequence encoding a frataxin protein, and conservatively modified variants thereof; (4) have a nucleic acid sequence that has greater than about 90%, preferably greater than about 91%, 92%, 93%, 94%, 95%, 96%, 97%, 98%, 99%, or higher nucleotide sequence identity, preferably over a region of at least about 25, 50, 100, 200, 500, 1000, 2000 or more nucleotides, or over the full-length, to a frataxin nucleic acid.

The term "Friedreich' s ataxia" and "FRDA" interchangeably to an autosomal recessive congenital ataxia caused by a mutation in gene FXN (formerly known as X25) that codes for frataxin, located on chromosome 9. The genetic basis for FRDA involves GAA trinucleotide repeats in an intron region of the gene encoding frataxin. This segment is normally repeated 5 to 33 times within the FXN gene. In people with Friedreich ataxia, the GAA segment is repeated 66 to more than 1,000 times. People with GAA segments repeated fewer than 300 times tend to have a later appearance of symptoms (after age 25) than those with larger GAA trinucleotide repeats. The presence of these repeats results in reduced transcription and expression of the gene. Frataxin is involved in regulation of mitochondrial iron content. The mutation in the FXN gene causes progressive damage to the nervous system, resulting in symptoms ranging from gait disturbance to speech problems; it can also lead to heart disease and diabetes. The ataxia of Friedreich's ataxia results from the degeneration of nerve tissue in the spinal cord, in particular sensory neurons essential (through connections with the cerebellum) for directing muscle movement of the arms and legs. The spinal cord becomes thinner and nerve cells lose some of their myelin sheath (the insulating covering on some nerve cells that helps conduct nerve impulses). A subject with FRDA may exhibit one or more of the following symptoms: muscle weakness in the arms and legs, loss of coordination, vision impairment, hearing impairment, slurred speech, curvature of the spine (scoliosis), high plantar arches (pes cavus deformity of the foot), carbohydrate intolerance, diabetes mellitus, heart disorders (e.g., atrial fibrillation, tachycardia (fast heart rate) and hypertrophic cardiomyopathy). A subject with FRDA may further exhibit involuntary and/or rapid eye movements, loss of deep tendon reflexes, loss of extensor plantar responses, loss of vibratory and proprioceptive sensation, cardiomegaly, symmetrical hypertrophy, heart murmurs, and heart conduction defects. Pathological analysis may reveal sclerosis and degeneration of dorsal root ganglia, spinocerebellar tracts, lateral corticospinal tracts, and posterior columns.

"Administering" refers to local or systemic administration, e.g., including enteral or parenteral administration. Routes of administration for the active agents that find use in the present invention include, e.g., oral ("po") administration, administration as a suppository, topical contact, intravenous ("iv"), intraperitoneal ("ip"), intramuscular ("im"), intralesional, intranasal, or subcutaneous ("sc") administration, or the implantation of a slow-release device e.g., a mini-osmotic pump, a depot formulation, and so forth, to a subject. Administration can be by any route including parenteral and transmucosal (e.g., oral, nasal, vaginal, rectal, or transdermal). Parenteral administration includes, e.g., intravenous, intramuscular, intra-arteriole, intradermal, subcutaneous, intraperitoneal, intraventricular, ionophoretic and intracranial. Other modes of delivery include, but are not limited to, the use of liposomal formulations, intravenous infusion, transdermal patches, and so forth The terms "systemic administration" and "systemically administered" refer to a method of administering a compound or composition to a mammal so that the compound or composition is delivered to sites in the body, including the targeted site of pharmaceutical action, via the circulatory system. Systemic administration includes, but is not limited to, oral, intranasal, rectal and parenteral (i.e., other than through the alimentary tract, such as intramuscular, intravenous, intra- arterial, transdermal and subcutaneous) administration.

The term "co-administer" and "co-administering" and variants thereof refer to administration of two active agents proximate in time to one another (e.g., within the same day, or week or period of 30 days, or sufficiently proximate that both drugs can be simultaneously detected in the blood, or otherwise sufficiently proximate that a synergistic effect results from the combined administration). An effect is considered synergistic if a more favorable response and/or fewer side effects are obtained from the co-administration of two (or more) agents than from administration of the same dose of each individual agent as the dose of the combined agent (dose can be measured as moles, moles/kg, mg or mg/kg). For example, co-administration of active agents A and B is considered synergistic if coadministration of 0.5 x moles A and 0.5 x moles B gives a better efficacy and/or reduced side effects than the separate administration of 1.0 X moles A and the separate administration of 1.0 x moles B. When co-administered, two or more active agents can be co-formulated as part of the same composition or administered as separate formulations.

PRKD1, also known as Protein Kinase D1, NPKC-Mu, PKD, EC 2.7.11.13, PRKCM, PKC-Mu, PKCM, PKC-MU, Protein Kinase C, Mu, Serine/Threonine-Protein Kinase D1, Protein Kinase C Mu Type, PKD1, Protein Kinase D, EC 2.7.11 or NPKC-D1, is a serine/threonine kinase that regulates a variety of cellular functions, including membrane receptor signaling, transport at the Golgi, protection from oxidative stress at the mitochondria, gene transcription, and regulation of cell shape, motility, and adhesion. The protein kinase D (PKD) family of serine/threonine protein kinases contains three members; PKD1, PKD2 and PKD3. These enzymes occupy a unique position in the signal transduction pathway initiated by diacylglycerol (DAG) and protein kinase C (PKC). PKDs are direct targets of DAG, and also lie downstream of PKC in a novel signal transduction pathway that is implicated in a variety of biological processes. Structurally, PKDs contain an N' terminal cysteine-rich domain (CRD), which binds phorbol esters with high affinity and has a role in mediating PKD translocation to the plasma membrane upon activation. The PH domains have an autoregulatory phosphorylation site (PKD1 and PKD2 only) and the catalytic domains have a high degree of homology to myosin light chain and calmodulin-dependent kinases. In the inactivated state, PKD1 and PKD2 are localized mainly to the cytoplasm, whilst PKD3 is found both in the cytoplasm and nucleus. Kinase activity of these enzymes is repressed by their CRD and PH domains. PKDs are activated by a variety of stimuli including regulatory peptides, lysophosphatidic acid, thrombin, PDGF, IGF-1, oxidative stress, cholecystokinin, Gbetagamma, ATP and more. These stimuli produce a rapid generation of DAG, which induces CRD-mediated PKD translocation from the cytosol to the plasma membrane. Novel PKCs are also recruited to the plasma membrane in response to DAG generation. Novel PKCs are allosterically activated by DAG, and transphosphorylate PKDs. This stabilizes PKD in its active conformation. Activated PKD dissociates from the plasma membrane, translocates to the cytosol and subsequently to the nucleus. PKDs have been implicated in fundamental physiological processes including signal transduction, membrane trafficking, and cell survival, migration, differentiation and proliferation. PKD upregulates the ERK and Ras signaling pathways, and suppresses the JNK signaling pathway. These enzymes regulate the budding of secretory vesicles from the trans-Golgi network and promote integrin recruitment to focal adhesions. In addition, PKDs have a role in regulating apoptosis and have functions in cell survival pathways induced by oxidative stress. They also have a role in immune regulation. Despite the plethora of physiological processes PKDs are involved in, only a few direct targets are known. These include kidins220, an integral membrane protein of neuroendocrine cells, c-Jun and RIN1, a protein that associates with Ras and 14.3.3 and activates the Ras-MEK-ERK pathway.

PRKD1 inhibitors are known in the art. Examples thereof are e.g. CID 755673 (George et al. Pharmaceutics, 2011 June 3(2)), [2,6] Naphtyhyridine (WO-A-2008/122615), bipyridyls as described in WO2009/150230, azols as described in WO2011/009484, antibody, or fragment thereof, specifically binding to protein kinase D1 and inhibiting its activity, siRNA down-regulating the expression of *PRKD1,* kb-NB142-70 (George et al. Pharmaceutics, 2011 June 3(2)), kb-NB165-09(George et al. Pharmaceutics, 2011 June 3(2)), and kmg-NB4-23 (George et al. Pharmaceutics, 2011 June 3(2)). Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

### Examples

**Cell culture.** Dermal fibroblasts from healthy, unaffected patients (WT, GM08399) and from patients affected by Friedreich's Ataxia (FRDA, GM04078) were obtained from Coriell Cell Repositories (Camden, N.J.). H9 hESCs were obtained from WiCell (Madison, Wis.). GM04078 cells were immortalized by lentiviral delivery of BMI1 and hTERT as described previously (Duss, S. et al. Breast Cancer Res 9, R38 (2007)), resulting in the cell line FRDA-4078iBT. GM04078 and FRDA-4078iBT human dermal fibroblasts (HDF) were maintained in MEM-α (Life Technologies, Grand Island, N.Y.) media containing 20% heat-inactivated FBS (Geminibio, West Sacramento, Calif.) and 1X L-glutamine (Life Technologies) or in Minimal Essential Medium Eagle (Sigma, St. Luis, Mo.) supplemented with 10-15% FCS (Sigma) and 2 mM L-glutamine at 37°C in 5% CO₂. HEK293T cells were cultured in Dulbecco's Modified Eagle Medium (DMEM) supplemented with 10% FCS (Sigma), 100 U ml⁻¹ penicillin, 100 pg ml⁻¹ streptomycin and 2 mM L-glutamine at 37°C in 5% CO₂. Human induced pluripotent stem cells (hiPSC) and human embryonic stem cells (hESC) were maintained in mTeSR (Stem Cell Technologies, B.C., Canada) on Growth-Factor-Reduced Matrigel (BD, San Jose, Calif.) culture conditions and were passaged every 4-6 days using dispase (Stem Cell Technologies). Karyotype and fingerprint analyses were performed by Cell Line Genetics (Madison, Wis.).

**Genome editing.** Plasmids encoding FXN-ZFN-L, FXN-ZFN-R, and donor constructs were co-transfected into FRDA-4078iBT or HEK293T cells by Amaxa Nucleofection (Lonza). Briefly, exponentially growing cells were trypsinized and 10⁶ cells harvested for transfection. Cells were resuspended in 100 µl Nucleofector Solution R, mixed with three non-linearized plasmids (two ZFN plasmids + Donor1 or Donor2) in a 1:10 molar ratio (ZFNs:Donor). Electroporation was performed in 5-10 µl nuclease-free water using program X-01 (Amaxa Nucleofector I, Lonza). Following transfection, cells were transferred to the respective pre-warmed media in 100-mm tissue culture dishes and cultured under standard conditions for 3 days prior to selection with puromycin or G418. Single puromycin- or G418-resistant clones were picked and expanded for further analysis.

**Immunoflorescent staining.** hiPSCs were seeded on a matrigel-coated black-clear bottom imaging plate (BD). Two days after plating, cells were washed once with PBS and fixed in 4% formaldehyde (USB Corp, Cleveland, Ohio)) for 20 min. Cells were permeabilized and incubated for 30 min in blocking buffer containing 3% Donkey Serum (Jackson Labs, Bar Harbor, Maine) and 0.1% Triton (Sigma) in PBS. Cells were stained with primary antibodies Oct4/Nanog (Abcam, Cambridge, Mass./Santa Cruz Biotech, Santa Cruz, Calif.) and dye-conjugated antibodies SSEA-4 and Tra-1-81/1-60 (BD) overnight at 4°C in blocking buffer. Cells were washed in 1% Tween (Sigma) in PBS and stained with secondary antibodies for 1 h at room temperature. Images were captured using a Zeiss Confocal Microscope (Thornwood, N.Y.).

**Luciferase assays.** Luciferase assays were performed with the Promega Dual-Luciferase Reporter Assay system according to the manufacturer's protocol. Briefly, lysis buffer was added directly to confluent cell monolayers. Culture dishes were incubated at 25°C for 15 min and rocked several times to ensure complete lysis. Cell lysates were centrifuged at maximum speed in an Eppendorf microcentrifuge for 10 min at 4°C. Luciferase measurements were performed with lysate supernatants in duplicate. All measurements were performed on a Centro LB 960 luminometer (Berthold Technologies, Germany). Bradford assays were carried out to determine total protein concentration, which was used for normalization.

**Pyrosequencing.** Cells were collected and outsourced to EpigenDX (Worcester, Mass.). Pyrosequencing analysis was performed according to standard procedures with primers that were developed for the CpG sites at positions (-50; +96) from the start codon of OCT4 (Assay ID: ADS510) gene.

**Real-time RT-PCR.** For quantitative RT-PCR (qRT-PCR) experiments, total RNA was extracted from GM04078, FRDA-4078iBT, and HEK293T-FF2AP cell lines with the Absolutely RNA Microprep Kit (Stratagene). A 1-µg aliquot of total RNA was reverse transcribed with AffinityScript enzyme (Stratagene) using random hexamers according to the manufacturer's instructions. qRT-PCR was performed on a CFX96 Real-Time PCR System (Bio-Rad) using the SsoAdvanced SYBR Green Supermix (Bio-Rad, #172-5264). Relative RNA levels were calculated from C_{T} values according to the ΔC_{T} method and normalized to GAPDH mRNA or 18S rRNA levels. Reprogrammed cells were washed once and total RNA isolated using TRIzol (Life Technologies). After chloroform extraction, RNA was purified using a RNeasy Plus Kit (Qiagen, Germantown, Md.). RNA purity and concentration were verified using an Aligent 2100 Bioanalyzer (Santa Clara, Calif.). In a two-step procedure, 1.5 µg of total RNA input was first used to synthesize cDNA with random primers with a High-Capacity cDNA Reverse Transcription Kit (Life Technologies) according to the manufacturer's instructions. Q-PCR analysis was performed on a ViiA-7 Real Time PCR System (Life Technologies) using Fast Advanced SYBR Green PCR master mix (Life Technologies). PCR conditions were as follows: 95°C - 5 min; (60°C - 30 s; x 40 cycles); 74°C - 10 min.

**RNAi screen.** A synthetic siRNA library targeting the druggable genome (4835 genes with 9670 constructs or two constructs per gene) was screened in duplicate 384-well plate format with two gene-targeting siRNAs per well to identify genes that upregulate frataxin-luciferase fusion expression. The library was reverse transfected into the HEK293T-FF2AP reporter cell line via high-throughput transfection (Aza-Blanc, P. et al. Molecular cell 12, 627-637 (2003); Chanda, S.K. et al. Proceedings of the National Academy of Sciences of the United States of America 100, 12153-12158 (2003)) using a fully integrated genomic robotic system (GNF Systems). Each plate contained transfection controls targeting cell viability (AllStars Cell Death, Qiagen), non-targeting (AllStars Negative Control, Qiagen) and a frataxin-targeting siRNA (Frataxin-12, Qiagen). Data from each plate were normalized to the plate mean and processed for fold activity. Duplicate readings for each well were geometrically averaged to generate a single activity score. A non-linear transformation was applied to remove plate variation (Konig, R. et al. Nature methods 4, 847-849 (2007)). Putative hit wells were rescreened as single siRNAs per well for validation. In addition to the first-round hit siRNAs, all siRNA constructs targeting genes of interest from other available libraries (Qiagen, Integrated DNA Technologies) were included in the validation screen. An additional 96 non-targeting controls were spotted into each 384-well plate of the validation assay (Qiagen). Data analysis included evaluation of individual and multiple well RNAi activities for each gene (Redundant siRNA Activity analysis) (Konig, R. et al. Nature methods 4, 847-849 (2007)).

**Sendai virus reprogramming and isolation.** Sendai virus (SeV) particles encoding OCT4, SOX2, KLF4 and cMYC (OSKM) were purchased from Life Technologies and SeV-reprogramming was performed as follows: patient and unaffected HDFs were seeded on Matrigel-coated 24-well plates at a density of 25-50 x 10³ per well. Cells were transduced with SeV particles encoding the 4-factors (MOI; O=10, SKM=7.5) overnight in fibroblast media containing 10 µg/ml polybrene (Sigma). The medium was changed to mTeSR 3 days post-infection. On days 5 or 6, cells were dissociated using accutase (Life Technologies) and plated onto Matrigel-coated 35-mm dishes seeded with mitomycin-C-treated human neonatal fibroblast feeders (GlobalStem, Rockville, Md.). Emerging hiPSC colonies were observed 20-30 days post-infection, selected and transferred to matrigel and mTeSR culture conditions.

**shRNA delivery.** shRNA constructs were delivered to FRDA-4078iBT cells by lentiviral transduction. Lentivirus was produced by calcium phosphate transfection of HEK293T cells (Dull, T. et al. Journal of virology 72, 8463-8471 (1998)). To transduce FRDA-4078iBT cells with multiple shRNA constructs, infections were performed simultaneously with different viruses. All infections were performed at a multiplicity of infection of approximately 30 viral particles per cell. Lipofection was used to deliver shRNA constructs into HEK293T-FF2AP cells; ∼200 cells per mm² were seeded 1 day before transfection. Cells were transfected with plasmids using Lipofectamine 2000 (Invitrogen) according to the manufacturer's protocol.

**Tri-layer differentiation and teratoma formation.** *Endoderm:* Accutase was used for cell dissociation to single cells, which were then plated on matrigel in mTeSR with rock-inhibitor Y27632 (Calbiochem, Darmstadt, Germany) at a density of 100 x 10³ per cm². Three days post-plating, the medium was changed to RPMI containing 0.5% FBS (Thermo, Waltham, Mass.) and 100 ng/ml Activin A (R&D, Minneapolis, Minn.). Cells were washed once, replenished with fresh media daily, fixed on day 5-6, and stained with Sox17 (Abcam) for analysis. *Mesoderm:* hiPSC/hESC cultures were grown to confluence. Using dispase (Stem Cell Technologies), cells were collected and plated into non-adherent 6-well culture dishes for embryoid body (EB) formation in media containing BMP4, G-CSF (R&D) and hSCF, hFlt3, and IL-3/6 (Peprotech, Rocky Hill, N.J.). Media were changed every 3 days. Cells were collected or re-plated on MG-coated 96-well plates for fixation at day 18 and then stained with α-Actinin (Abcam) for analysis. *Ectoderm and neural derivatives*: Undifferentiated hESC/hiPSC cultures were first exposed to dual inhibition of SMAD signaling, which has been shown to convert hESC to neural progenitors (Chambers, S.M. et al. Nat Biotechnol 27, 275-280 (2009)). For neuronal differentiation, neural progenitors were expanded in DMEM/F-12 with B27 and N2 supplements (Gibco), 10 ng/ml human epidermal growth factor (hEGF) and 10 ng/ml human basic fibroblast growth factor (hbFGF) (Gibco). Neural progenitors were finally differentiated into neurons in neurobasal medium (Gibco) supplemented with B27, 10 ng/ml human neurotrophin-3 (hNT-3) (R & D), and 10 ng/ml human brain-derived neurotrophic factor (hBDNF) (R & D) for at least 4 weeks. Antibodies against Pax6 (DSHB) and β-III Tubulin (Tuj1, Covance) were used to identify neural progenitors and neurons, respectively. In order to quantify the efficiency of differentiation into Pax6-expressing neural progenitors, immunostained cultures were generated and the percentage of Pax6-positive and DAPI-positive cells determined in 10 randomly selected microscopic fields. In order to quantify the efficiency of differentiation into β-III Tubulin-expressing neurons, a similar approach was used with cultures previously treated with cytosine-1-β-D-arabinofuranoside to prevent the expansion of neural progenitors. *Teratoma formation* was outsourced to Applied Stem Cells (Menlo Park, Calif.). **Western blotting.** Protein samples were separated on NuPAGE-Novex Bis-Tris 4-12% gradient gels (Invitrogen) in MES buffer at 200 V for 40 min. Transfer to optitran nitrocellulose membrane BA-S 85 (Whatman) was performed at 60 mA (constant) overnight in transfer buffer with 20% methanol. The membranes were blocked in 5% non-fat dry milk in TBST for at least 30 min at room temperature and subsequently washed once in TBST for 5-10 min. The membranes were incubated with the anti-FXN antibody (SKU#456300, Invitrogen) in 5% non-fat dry milk in TBST overnight at 4°C. Membranes were washed in TBST and incubated with secondary antibody (anti-mouse HRP, 1:5000) in 5% non-fat dry milk in TBST for 45-60 min at room temperature. Blots were developed with Immobilon Western HRP Substrate (Millipore).

To address the needs in the art, the inventors aimed first at generating a reporter cell line of human origin that is compatible with high-throughput biology and enables accurate monitoring of endogenous FXN gene expression. To this end, they chose a zinc-finger nuclease (ZFN)-mediated genome-editing approach to tag the 3' end of the endogenous FXN gene with a firefly luciferase (FL) reporter gene (Lombardo, A. et al. Nature biotechnology 25, 1298-1306 (2007); Urnov, F.D. et al. Nature 435, 646-651 (2005)). To induce a double-strand break approximately 1 kb downstream of the 5' splice site in the last intron of the FXN gene, the inventors designed a pair of ZFNs that bind to the FXN locus uniquely. For homologous recombination, they created a transgenic donor DNA fragment encoding the last FXN exon fused to the open reading frame of FL. To allow clonal selection after targeted insertion, they also created donor constructs encoding either puromycin N-acetyl-transferase (Puro) preceded by 2A self-cleaving peptide linked to the FXN-FL fusion (Donor-1) or an autonomous neomycin (Neo) selection cassette (Donor-2). The left homology arm of the donor constructs was designed to introduce a functional branchpoint and 3' splice sites to favor efficient splicing between exon 4 and inserted exon 5. The inventors used this ZFN system to introduce a luciferase reporter into the FXN locus of human embryonic kidney (HEK) 293T cells. This cell line was selected because it can be expanded to a very large scale at a relatively low cost. As a consequence, their FXN reporter system is thought to be compatible with the technical and financial constraints of most academic and industrial screening platforms. The inventors co-transfected human HEK 293T cells with expression plasmids encoding the two frataxin-ZFNs (ZFN-L and ZFN-R) and a plasmid encoding Donor-1, which confers puromycin resistance only after correct integration, proper pre-mRNA splicing and translation of the FXN-FL-2A-PURO (FF2AP) fusion protein. At 2-3 weeks post-transfection, puromycin-resistant colonies were isolated and subjected to western blot analysis. This revealed specific expression of a 75-kDa FXN-FL fusion protein in puromycin-resistant cells (hereafter referred to as HEK293T-FF2AP. The inventors observed the same molecular weight when the FXN-FL fusion protein was expressed ectopically from a plasmid, demonstrating efficient co-translational self-cleavage of the 2A peptide and release of the Puro protein. Importantly, they detected firefly luciferase activity in lysates from HEK293T-FF2AP but not from parental HEK293T cells, an activity that could be abrogated upon expression of a short-hairpin RNA (shRNA) targeting exon 2 of the FXN mRNA. Thus, they concluded that the firefly luciferase activity in HEK293T-FF2AP cells came solely from the targeted integration at the *FXN* locus and, thus, reliably reports expression of the FXN gene from its endogenous location.

In contrast to previous work (Martelli, A., Napierala, M. & Puccio, H. Dis Model Mech 5, 165-176 (2012)), the HEK293T-FF2AP cell line allowed the present inventors to screen for modulators of FXN expression in its natural genomic context and in an HTS format. This cell line is easy to transfect and hence well suited for RNA interference (RNAi) or cDNA overexpression screens. Therefore, to identify potential repressors of FXN expression, they performed an RNAi screen targeting 4,835 human genes in the HEK293T-FF2AP cell line, using luciferase signal amplification as the readout. Two unique siRNAs were assayed in a pooled format at 72 h post-transfection. Due to the pooled construct format, they chose a baseline of 1.6-fold luciferase activation over the mean as a cut-off in order to capture phenotypes generated from wells in which only one siRNA may be active. In subsequent validation screens, positive hit siRNAs were spotted in an arrayed well format of 1 siRNA per well and screened in duplicate under identical conditions. Using the HEK293T-FF2AP cell line, the inventors performed a reconfirmation screen of the primary screen hits. In parallel, they screened positive-hit siRNAs for non-FXN-specific activators of luciferase activity with an HEK 293T cell line stably expressing luciferase from a CMV promoter (HEK293-pGF1-CMV) (ref). This allowed for thorough analysis of independent constructs via redundant siRNA analysis (RSA) (Konig, R. et al. Nature methods 4, 847-849 (2007)), which enriched for genes where two or more independent siRNAs elevated the FXN-luciferase protein levels specifically. The FXN alleles in the HEK293T-FF2AP cell line used in the primary RNAi screen do not contain pathogenic GAA triplet repeat expansions. The present inventors designed the screening cell line that way because high-throughput biology requires large amounts of cells and FXN deficiency could prevent cell expansion. In order to determine whether the primary hits could increase FXN gene expression in cells that contain an expanded repeat, they performed secondary hit validation experiments in immortalized FRDA-patient fibroblasts (FRDA-4078iBT). Expression analysis revealed that five candidate genes are also expressed in FRDA fibroblasts (PRKD1, CDK12, CDK13, SBF1, and NR1H3). To knock down these genes, three different shRNA constructs per candidate gene were designed and delivered into fibroblasts by lentiviral transduction. Except for SBF1, mRNA levels of all candidate genes were reduced upon shRNA expression. Depletion of the cell cycle related kinases CDK12 and CDK13 resulted in strong proliferation defects and were omitted from further analysis. Even though knockdown efficiencies for PRKD1 or NR1H3 were never higher than 60%, the inventors consistently observed a significant increase in FXN mRNA levels. These results confirmed that reducing the levels of the serine/threonine-protein kinase PRKD1 or the nuclear receptor NR1H3 results in increased FXN expression even in the presence of an expanded repeat tract. It is noteworthy that previous attempts to identify FXN repressors with artificial reporter constructs identified neither PRKD1 nor NR1H3. This result confirms that monitoring gene expression from the endogenous FXN locus opens up new opportunities for target discovery and the dissection of molecular pathways involved in FRDA.

The inventors completed our drug discovery platform by designing disease-relevant models, which not only validate primary hits as druggable targets but also enable the discovery of chemical modulators of these targets. They first reprogrammed dermal fibroblasts from healthy, unaffected individuals (wildtype WT, GM08399) and from patients affected by FRDA (FRDA, GM04078) into induced pluripotent stem cells (iPSCs). Sendai virus particles encoding OCT4, SOX2, KLF4, and cMYC (OSKM) were used in order to minimize the risk that random integration of reprogramming transgenes would affect FXN pathways. The iPSCs obtained from both WT and FRDA patients and all iPSC lines expressed similar pluripotency cell markers. The iPSC lines could also differentiate in representatives of all three germ layers, including neurons and cardiomyocytes, two cell types that are affected in FRDA patients. Both WT and FRDA iPSCs also formed teratomas when injected into mice. Importantly, the iPSC lines obtained from WT and FRDA patients exhibited a similar ability to differentiate into Pax6-expressing neural progenitors and β-III Tubulin-expressing neurons. Finally, FRDA neuronal cells expressed lower levels of FXN mRNA and protein than WT, indicating that the FRDA molecular phenotype is conserved during reprogramming.

In order to identify low molecular weight modulators of PRKD1, a compound archive was searched. As disclosed in WO-A-2008/122615, 2,6-naphthyridine had been identified as a dual PKC/PRKD inhibitor that was further developed into a potent prototype pan-PRKD inhibitor. Rational design and modification led to the [2,6] Naphtyhyridine is the cyclohexyl-[4-(4-phenyl-1-piperazin-1-yl-[2,6]naphthyridin-3-yl)pyridin-2-yl]amine of formual (II):

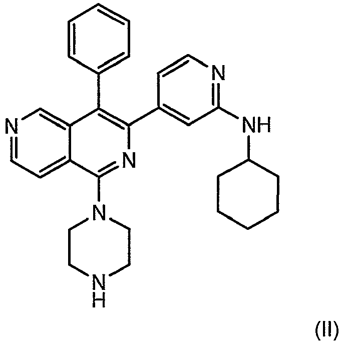

FRDA-iPSC-derived neurons were treated with either the compound of formula (II) or a non-2,6-naphthyridine control compound but lacks activity on PRKD1. As observed with shRNA-mediated suppression in fibroblasts, chemical inhibition of PRKD1 by the compound of formula (II) resulted in a significant increase in endogenous FXN gene expression in FRDA-iPSC-derived neurons. Importantly, the control compound had no significant effect on FXN expression in FRDA patient neurons, indicating that activity of the compound of formula (II) is due to an inhibitory effect on PRKD1. Importantly, treatment with the compound of formula (II) led to a 25% increase in FXN mRNA expression in FRDA neurons after only 3 weeks of treatment. Because many FRDA patients exhibit FXN levels which are only 20-30% lower than asymptomatic carriers, a 25% increase in FXN expression is expected to be therapeutically sufficient for at least a subpopulation of FRDA patients (Miranda, C.J. et al.. FEBS Lett 512, 291-297 (2002); Martelli, A., Napierala, M. & Puccio, H. Dis Model Mech 5, 165-176 (2012)). In addition, neuron viability was not affected by exposure to the compound of formula (II), even at 10 µM.

## Claims

1. An agent which is a modulator of the activitiy of the *PRKD1* gene product, protein kinase D1, for use in the treatment of Friedreich's ataxia, wherein said agent is a protein kinase D1 inhibitor and is a [2,6] Naphthyridine of formula (I): 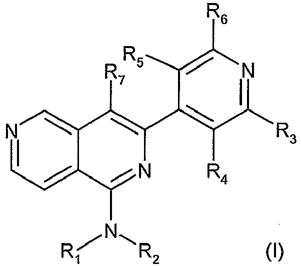 wherein
R₁ and R₂ are independently hydrogen, alkyl, cycloalkyl, heterocyclyl, each of which is optionally substituted by one to two R₈, wherein R₈ is hydrogen, halogen, alkyl, R₉-O--, (R₁₀)(R₁₁)N--, (R₁₂)(R₁₃)N-C(O)--, aryl, or heterocyclyl or heteroaryl, said heterocyclyl and heteroaryl are optionally substituted by one or two alkyl groups;
R₁ and R₂ taken together with the nitrogen atom to which they are attached to optionally form a 4-7 membered ring;
R₃ is (R₁₄)(R₁₅)N--, or halogen;
R₄, R₅, R₆ and R₇ are independently hydrogen, halogen, alkyl, (C₃-C₇) cycloalkyl, aryl-alkyl, aryl, or alkoxy;
R₉, R₁₀, R₁₁, R₁₂ and R₁₃ are independently hydrogen, alkyl-O-C(O)--, alkyl-NH-C(O)--, alkyl-C(O)-NH-C(O)--, cycloalkyl, cycloalkyl-alkyl--, R₁₆-SO₂--, R₁₇-C(O)--, heterocyclyl or alkyl, said heterocyclyl is further optionally substituted by one or two cycloalkyl-alkyl-- groups, and said alkyl is further optionally substituted by one or two groups selected from hydroxy, alkoxy, alkylamine, dialkylamine, or heteroaryl;
R₁₀ and R₁₁ taken together with the nitrogen atom to which they are attached to optionally form a 5-7 membered ring;
R₁₂ and R₁₃ taken together with the nitrogen atom to which they are attached to optionally form a 5-7 membered ring;
R₁₄ and R₁₅ are independently hydrogen, alkyl, aryl, cycloalkyl, aryl-alkyl--, heterocyclyl or heteroaryl, said alkyl, cycloalkyl, aryl and heteroaryl are further optionally substituted by one or two groups selected from alkyl, alkoxy, hydroxy, halogen, haloalkyl, cyano, or R₁₈-NH-C(O)--;
R₁₆ is aryl or heteroaryl;
R₁₇ is heterocyclyl, or alkyl optionally substituted by one or two groups selected from H₂N--, aryl-alkyl--, or alkyl-C(O)-NH--;
R₁₈ is heterocyclyl-alkyl--; or
a pharmaceutically acceptable salt thereof; or an optical isomer thereof; or a mixture of optical isomers.

2. The agent for use of claim 1 wherein said [2,6] Naphthyridine is according to Formula I wherein R₁ and R₂ are independently hydrogen, (C₁-C₇)alkyl, (C₃-C₇)cycloalkyl, heterocyclyl, each of which is optionally substituted by one to two R₈, wherein R₈ is hydrogen, halogen, (C₁-C₇)alkyl, R₉-O--, (R₁₀)(R₁₁)N--, (R₁₂)(R₁₃)N-C(O)--, (C₆-C₁₀)aryl, or heterocyclyl or heteroaryl, said heterocyclyl and heteroaryl are optionally substituted by one or two (C₁-C₇)alkyl groups;
R₁ and R₂ taken together with the nitrogen atom to which they are attached to optionally form a 4-7 membered ring;
R₃ is (R₁₄)(R₁₅)N;
R₄, R₅, R₆ are H; and
R₇ is independently hydrogen, halogen, (C₁-C₇)alkyl, (C₃-C₇)cycloalkyl, (C₆-C₁₀)aryl-(C₁-C₇)alkyl, (C₆-C₁₀)aryl, or (C₁-C₇)alkoxy;
R₉, R₁₀, R₁₁, R₁₂ and R₁₃ are independently hydrogen, (C₁-C₇)alkyl-O-C(O)--, (C₁-C₇)alkyl-NH-C(O)--, (C₁-C₇)alkyl-C(O)-NH-C(O)--, (C₃-C₇)cycloalkyl, (C₃-C₇)cycloalkyl-(C₁-C₇)alkyl-, R₁₆-SO₂--, R₁₇-C(O)--, heterocyclyl or (C₁-C₇)alkyl, said heterocyclyl is further optionally substituted by one or two (C₃-C₇)cycloalkyl-(C₁-C₇)alkyl-- groups, and said alkyl is further optionally substituted by one or two groups selected from hydroxy, (C₁-C₇)alkoxy, (C₁-C₇)alkylamine, di-(C₁-C₇)alkylamine, or heteroaryl;
R₁₀ and R₁₁ taken together with the nitrogen atom to which they are attached to optionally form a 5-7 membered ring;
R₁₂ and R₁₃ taken together with the nitrogen atom to which they are attached to optionally form a 5-7 membered ring;
R₁₄ and R₁₅ are independently hydrogen, (C₁-C₇)alkyl, (C₆-C₁₀)aryl, (C₃-C₇)cycloalkyl, (C₆-C₁₀)aryl-(C₁-C₇)alkyl--, heterocyclyl or heteroaryl, said alkyl, cycloalkyl, aryl and heteroaryl are further optionally substituted by one or two groups selected from (C₁-C₇)alkyl, (C₁-C₇)alkoxy, hydroxy, halogen, halo(C₁-C₇)alkyl, cyano, or R₁₈-NH-C(O)--;
R₁₆ is aryl or heteroaryl;
R₁₇ is heterocyclyl, or (C₁-C₇)alkyl optionally substituted by one or two groups selected from H₂N--, (C₆-C₁₀)aryl-(C₁-C₇)alkyl--, or (C₁-C₇)alkyl-C(O)-NH--;
R₁₈ is heterocyclyl-(C₁-C₇)alkyl--; or
wherein heterocyclyl refers to an optionally substituted, saturated or unsaturated non-aromatic ring or ring system, which is a 4-, 5-, 6-, or 7-membered monocyclic; and
heteroaryl refers to a 5-14 membered monocyclic- or bicyclic- or polycyclic-aromatic ring system, having 1 to 8 heteroatoms selected from N, O or S;
a pharmaceutically acceptable salt thereof; or an optical isomer thereof; or a mixture of optical isomers

3. The agent for use of claim 2 wherein said [2,6] Naphthyridine is the cyclohexyl-[4-(4-phenyl-1-piperazin-1-yl-[2,6]naphthyridin-3-yl)pyridin-2-yl]amine of formula (II):

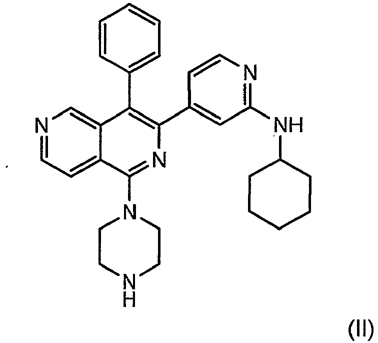

## Patentansprüche

1. Mittel, bei dem es sich um einen Modulator der Aktivität des *PRKD1*-Genprodukts, Proteinkinase D1, handelt, zur Verwendung bei der Behandlung von Friedreich-Ataxie, wobei das Mittel ein Proteinkinase-D1-Inhibitor ist und ein [2,6]-Naphthyridin der Formel (I) ist: 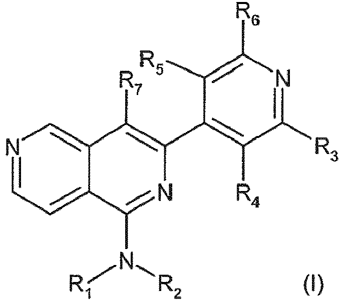 wobei
R₁ und R₂ unabhängig für Wasserstoff, Alkyl, Cycloalkyl oder Heterocyclyl stehen, die jeweils gegebenenfalls durch einen oder zwei Reste R₈ substituiert sind, wobei R₈ für Wasserstoff, Halogen, Alkyl, R₉-O--, (R₁₀)(R₁₁)N--, (R₁₂)(R₁₃)N-C(O)--, Aryl, Heterocyclyl oder Heteroaryl steht, wobei das Heterocyclyl und Heteroaryl gegebenenfalls durch eine oder zwei Alkylgruppen substituiert sind;
R₁ und R₂ zusammen mit dem Stickstoffatom, an das sie gebunden sind, gegebenenfalls einen 4-7-gliedrigen Ring bilden;
R₃ für (R₁₄)(R₁₅)N-- oder Halogen steht;
R₄, R₅, R₆ und R₇ unabhängig für Wasserstoff, Halogen, Alkyl, (C₃-C₇)-Cycloalkyl, Arylalkyl, Aryl oder Alkoxy stehen;
R₉, R₁₀, R₁₁, R₁₂ und R₁₃ unabhängig für Wasserstoff, Alkyl-O-C(O)--, Alkyl-NH-C(O)--, Alkyl-C(O)-NH-C(O)--, Cycloalkyl, Cycloalkylalkyl--, R₁₆-SO₂--, R₁₇-C(O)--, Heterocyclyl oder Alkyl stehen, wobei das Heterocyclyl gegebenenfalls weiter durch eine oder zwei Cycloalkylalkyl--Gruppen substituiert ist und das Alkyl gegebenenfalls weiter durch eine oder zwei Gruppen, die aus Hydroxy, Alkoxy, Alkylamin, Dialkylamin und Heteroaryl ausgewählt sind, substituiert ist;
R₁₀ und R₁₁ zusammen mit dem Stickstoffatom, an das sie gebunden sind, gegebenenfalls einen 5-7-gliedrigen Ring bilden;
R₁₂ und R₁₃ zusammen mit dem Stickstoffatom, an das sie gebunden sind, gegebenenfalls einen 5-7-gliedrigen Ring bilden;
R₁₄ und R₁₅ unabhängig für Wasserstoff, Alkyl, Aryl, Cycloalkyl, Arylalkyl--, Heterocyclyl oder Heteroaryl stehen, wobei das Alkyl, Cycloalkyl, Aryl und Heteroaryl ferner gegebenenfalls durch eine oder zwei Gruppen, die aus Alkyl, Alkoxy, Hydroxy, Halogen, Halogenalkyl, Cyano und R₁₈-NH-C(O)-- ausgewählt sind, substituiert sind, stehen; R₁₆ für Aryl oder Heteroaryl steht;
R₁₇ für Heterocyclyl oder Alkyl, das gegebenenfalls durch eine oder zwei Gruppen, die aus H₂N--, Arylalkyl-- und Alkyl-C(O)-NH-- ausgewählt sind, substituiert ist, steht;
R₁₈ für Heterocyclylalkyl-- steht; oder
ein pharmazeutisch unbedenkliches Salz davon; oder ein optisches Isomer davon; oder ein Gemisch von optischen Isomeren.

2. Mittel zur Verwendung nach Anspruch 1, wobei das [2,6]-Naphthyridin der Formel I entspricht, wobei
R₁ und R₂ unabhängig für Wasserstoff, (C₁-C₇)-Alkyl, (C₃-C₇)-Cycloalkyl oder Heterocyclyl stehen, die jeweils gegebenenfalls durch einen oder zwei Reste R₈ substituiert sind, wobei R₈ für Wasserstoff, Halogen, (C₁-C₇)-Alkyl, R₉-O--, (R₁₀)(R₁₁)N--, (R₁₂)(R₁₃)N-C(O)--, (C₆-C₁₀)-Aryl, Heterocyclyl oder Heteroaryl steht, wobei das Heterocyclyl und Heteroaryl gegebenenfalls durch eine oder zwei (C₁-C₇)-Alkylgruppen substituiert sind;
R₁ und R₂ zusammen mit dem Stickstoffatom, an das sie gebunden sind, gegebenenfalls einen 4-7-gliedrigen Ring bilden;
R₃ für (R₁₄) (R₁₅)N-- steht;
R₄, R₅, R₆ für H stehen; und
R₇ unabhängig für Wasserstoff, Halogen, (C₁-C₇)-Alkyl, (C₃-C₇)-Cycloalkyl, (C₆-C₁₀)-Aryl-(C₁-C₇)-alkyl, (C₆-C₁₀)-Aryl oder (C₁-C₇)-Alkoxy steht;
R₉, R₁₀, R₁₁, R₁₂ und R₁₃ unabhängig für Wasserstoff, (C₁-C₇)-Alkyl-O-C(O)--, (C₁-C₇)-Alkyl-NH-C(O)--, (C₁-C₇)-Alkyl-C(O)-NH-C(O)--, (C₃-C₇)-Cycloalkyl, (C₃-C₇)-Cycloalkyl-(C₁-C₇)-alkyl--, R₁₆-SO₂--, R₁₇-C(O)--, Heterocyclyl oder (C₁-C₇)-Alkyl stehen, wobei das Heterocyclyl gegebenenfalls weiter durch eine oder zwei (C₃-C₇)-Cycloalkyl-(C₁-C₇)-alkyl-Gruppen substituiert ist und das Alkyl gegebenenfalls weiter durch eine oder zwei Gruppen, die aus Hydroxy, (C₁-C₇)-Alkoxy, (C₁-C₇)-Alkylamin, Di-(C₁-C₇)-alkylamin und Heteroaryl ausgewählt sind, substituiert ist;
R₁₀ und R₁₁ zusammen mit dem Stickstoffatom, an das sie gebunden sind, gegebenenfalls einen 5-7-gliedrigen Ring bilden;
R₁₂ und R₁₃ zusammen mit dem Stickstoffatom, an das sie gebunden sind, gegebenenfalls einen 5-7-gliedrigen Ring bilden;
R₁₄ und R₁₅ unabhängig für Wasserstoff, (C₁-C₇)-Alkyl, (C₆-C₁₀)-Aryl, (C₃-C₇)-Cycloalkyl, (C₆-C₁₀)-Aryl-(C₁-C₇)-alkyl--, Heterocyclyl oder Heteroaryl stehen, wobei das Alkyl, Cycloalkyl, Aryl und Heteroaryl ferner gegebenenfalls durch eine oder zwei Gruppen, die aus (C₁-C₇)-Alkyl, (C₁-C₇)-Alkoxy, Hydroxy, Halogen, Halogen-(C₁-C₇)-alkyl, Cyano und R₁₈-NH-C(O)-- ausgewählt sind, substituiert sind, stehen;
R₁₆ für Aryl oder Heteroaryl steht;
R₁₇ für Heterocyclyl oder (C₁-C₇)-Alkyl, das gegebenenfalls durch eine oder zwei Gruppen, die aus H₂N--, (C₆-C₁₀)-Aryl-(C₁-C₇)-alkyl-- und (C₁-C₇)-Alkyl-C(O)-NH-- ausgewählt sind, substituiert ist, steht;
R₁₈ für Heterocyclyl-(C₁-C₇)-alkyl-- steht; oder wobei sich Heterocyclyl auf einen gegebenenfalls substituierten gesättigten oder ungesättigten nichtaromatischen Ring oder ein entsprechendes Ringsystem, wobei es sich um einen 4-, 5-, 6- oder 7-gliedrigen monocyclischen Ring handelt, bezieht; und
wobei sich Heteroaryl auf ein 5-14-gliedriges monocyclisches oder bicyclisches oder polycyclisches aromatisches Ringsystem mit 1 bis 8 Heteroatomen, die aus N, 0 oder S ausgewählt sind, bezieht;
ein pharmazeutisch unbedenkliches Salz davon; oder ein optisches Isomer davon; oder ein Gemisch von optischen Isomeren.

3. Mittel zur Verwendung nach Anspruch 2, wobei es sich bei dem [2,6]-naphthyridin um das Cyclohexyl-[4-(4-phenyl-1-piperazin-1-yl-[2,6]naphthyridin-3-yl)pyridin-2-yl]amin der Formel (II) handelt:

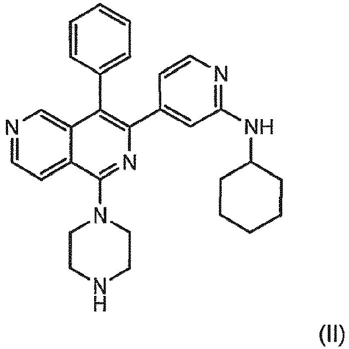

## Revendications

1. Agent qui est un modulateur de l'activité du produit génique *PRKD1,* protéine kinase D1, destiné à être utilisé dans le traitement de l'ataxie de Friedreich, ledit agent étant un inhibiteur de la protéine kinase D1 et étant une [2,6]Naphtyridine de formule (I) : 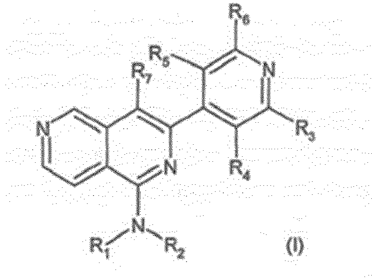 dans laquelle
R₁ et R₂ sont indépendamment hydrogène, alkyle, cycloalkyle, hétérocyclyle, dont chacun est substitué, en option, par un à deux R₈, dans laquelle R₈ est : hydrogène, halogène, alkyle, R₉-0--, (R₁₀)(R₁₁)N--, (R₁₂)(R₁₃)N-C(O)--, aryle ou bien hétérocyclyle ou hétéroaryle, lesdits hétérocyclyle et hétéroaryle sont substitués, en option, par un ou deux groupe(s) alkyle ;
R₁ et R₂ sont pris ensemble avec l'atome d'azote auquel ils sont fixés pour former, en option, un anneau de 4 à 7 membres ;
R₃ est (R₁₄) (R₁₅)N-- ou halogène ;
R₄, R₅, R₆ et R₇ sont indépendamment hydrogène, halogène, alkyle, cycloalkyle en (C₃-C₇), aryl-alkyle, aryle ou alcoxy ;
R₉, R₁₀, R₁₁, R₁₂ et R₁₃ sont indépendamment hydrogène, alkyl-O-C(O)--, alkyl-NH-C(O)--, alkyl-C(O)-NH-C(O)--, cycloalkyle, cycloalkyl-alkyl--, R₁₆-SO₂--, R₁₇-C(O)--, hétérocyclyle ou alkyle, ledit hétérocyclyle est, en option, en outre substitué par un ou deux groupe(s) cycloalkyl-alkyl-- et ledit alkyle est, en option, en outre substitué par un ou deux groupe(s) choisi(s) parmi hydroxy, alcoxy, alkylamine, dialkylamine ou hétéroaryle ;
R₁₀ et R₁₁ sont pris ensemble avec l'atome d'azote auquel ils sont fixés pour former, en option, un anneau de 5 à 7 membres ;
R₁₂ et R₁₃ sont pris ensemble avec l'atome d'azote auquel ils sont fixés pour former, en option, un anneau de 5 à 7 membres ;
R₁₄ et R₁₅ sont indépendamment hydrogène, alkyle, aryle, cycloalkyle, aryl-alkyl--, hétérocyclyle ou hétéroaryle, lesdits alkyle, cycloalkyle, aryle et hétéroaryle sont, en option, en outre substitués par un ou deux groupe(s) choisi(s) parmi : alkyle, alcoxy, hydroxy, halogène, haloalkyle, cyano ou R₁₈-NH-C(O)-- ;
R₁₆ est aryle ou hétéroaryle ;
R₁₇ est hétérocyclyle ou alkyle substitué, en option, par un ou deux groupe(s) choisi(s) parmi : H₂N--, aryl-alkyl-- ou alkyl-C(O)-NH-- ;
R₁₈ est hétérocyclyl-alkyl-- ; ou
sel de celui-ci acceptable d'un point de vue pharmaceutique ; ou isomère optique de celui-ci ; ou mélange d'isomères optiques.

2. Agent destiné à l'utilisation de la revendication 1, dans lequel ladite [2,6]naphtyridine est selon la Formule I, dans laquelle R₁ et R₂ sont indépendamment hydrogène, alkyle en (C₁-C₇), cycloalkyle en (C₃-C₇), hétérocyclyle, dont chacun est substitué, en option, par un à deux R₈, dans laquelle R₈ est hydrogène, halogène, alkyle en (C₁-C₇), R₉-O--, (R₁₀)(R₁₁)N--, (R₁₂)(R₁₃)N-C(O)--, aryle en (C₆-C₁₀) ou bien hétérocyclyle ou hétéroaryle, lesdits hétérocyclyle et hétéroaryle sont substitués, en option, par un ou deux groupe (s) alkyle en (C₁-C₇);
R₁ et R₂ sont pris ensemble avec l'atome d'azote auquel ils sont fixés pour former, en option, un anneau de 4 à 7 membres ;
R₃ est (R₁₄) (R₁₅)N ;
R₄, R₅, R₆ sont H ; et
R₇ est indépendamment hydrogène, halogène, alkyle en (C₁-C₇), cycloalkyle en (C₃-C₇), aryle en (C₆-C₁₀)-alkyle en (C₁-C₇), aryle en (C₆-C₁₀) ou alcoxy en (C₁-C₇);
R₉, R₁₀, R₁₁, R₁₂ et R₁₃ sont indépendamment hydrogène, alkyle en (C₁-C₇)-O-C(O)--, alkyle en (C₁-C₇)-NH-C(O)--, alkyle en (C₁-C₇)-C(O)-NH-C(O)--, cycloalkyle en (C₃-C₇), cycloalkyle en (C₃-C₇)-alkyle en (C₁-C₇)--, R₁₆-SO₂--, R₁₇-C(O)--, hétérocyclyle ou alkyle en (C₁-C₇), ledit hétérocyclyle est, en option, en outre substitué par un ou deux groupe (s) cycloalkyle en (C₃-C₇)-alkyle en (C₁-C₇)-- et ledit alkyle est, en option, en outre substitué par un ou deux groupe(s) choisi(s) parmi hydroxy, alcoxy en (C₁-C₇), alkylamine en (C₁-C₇), di-alkylamine en (C₁-C₇)ou hétéroaryle ;
R₁₀ et R₁₁ sont pris ensemble avec l'atome d'azote auquel ils sont fixés pour former, en option, un anneau de 5 à 7 membres ;
R₁₂ et R₁₃ sont pris ensemble avec l'atome d'azote auquel ils sont fixés pour former, en option, un anneau de 5 à 7 membres ;
R₁₄ et R₁₅ sont indépendamment hydrogène, alkyle en (C₁-C₇), aryle en (C₆-C₁₀), cycloalkyle en (C₃-C₇), aryle en (C₆-C₁₀) -alkyle en (C₁-C₇)--, hétérocyclyle ou hétéroaryle, lesdits alkyle, cycloalkyle, aryle et hétéroaryle sont, en option, en outre substitués par un ou deux groupe(s) choisi(s) parmi alkyle en (C₁-C₇), alcoxy en (C₁-C₇), hydroxy, halogène, halo-alkyle en (C₁-C₇), cyano ou R₁₈-NH-C(O)--;
R₁₆ est aryle ou hétéroaryle ;
R₁₇ est hétérocyclyle ou alkyle en (C₁-C₇) substitué, en option, par un ou deux groupe(s) choisi(s) parmi H₂N--, aryle en (C₆-C₁₀)-alkyle en (C₁-C₇)-- ou alkyle en (C₁-C₇)-C(O)-NH-- ;
R₁₈ est hétérocyclyl-alkyle en (C₁-C₇)-- ; ou
dans laquelle « hétérocyclyle » se réfère à un anneau ou un système d'anneau non aromatique, en option substitué, saturé ou insaturé, qui est monocyclique à 4, 5, 6 ou 7 membres ; et
« hétéroaryle » se réfère à un système d'anneau aromatique monocyclique, bicyclique ou polycyclique à 5 à 14 membres, ayant de 1 à 8 hétéroatomes choisi (s) parmi N, 0 ou S ;
sel de celui-ci acceptable d'un point de vue pharmaceutique ; ou un isomère optique de celui-ci ; ou mélange d'isomères optiques.

3. Agent destiné à l'utilisation de la revendication 2, dans lequel ladite [2,6]naphtyridine est la cyclohexyl-[4-(4-phényl-1-pipérazin-1-yl-[2,6]naphtyridin-3-yl)pyridin-2-yl]amine de formule (II) :
